# EUROPEAN PATENT APPLICATION

(11) **EP 4 471 020 A1**
(43) Date of publication of application: **04.12.2024**
(21) Application number: 23305849.4
(22) Date of filing: 30.05.2023
(51) Int. Cl.: C07D 335/16, C07C 49/84, C08K 5/45

(54) **CHROMOPHORE-FUNCTIONAL POLYOLS**

(71) Applicant: ARKEMA FRANCE, 92700 Colombes (FR)
(72) Inventor: HERR, Donald, EXTON, 19341 (US); HE, Yuhong, EXTON, 19341 (US)
(74) Representative: Arkema Patent

(57) **Abstract**

Polyols having at least one chromophore moiety are provided. The polyols are useful for preparing photocurable compositions that do not require an additional separate chromophore.

## Description

### FIELD OF THE INVENTION

Polyols having at least one chromophore moiety are provided. The polyols are useful for preparing photocurable compositions that do not require an additional separate chromophore.

### BACKGROUND

In light or other actinic radiation curable systems, including those that contain urethane (meth)acrylates, the radicals which initiate cure are created through the photodecomposition of a photoinitiator molecule. A wide variety of such small molecule photoinitiators are commercially available and well known to those skilled in the art. A drawback of using small molecule photoinitiators is that inevitably small molecule photofragments remain in the cured composition. This is true whether one uses fragmentation photoinitiators (Norrish Type I) or hydrogen abstraction bimolecular photoinitiators (Norrish Type II). Small molecule fragments are often the source of undesirable odor, volatile organic content, and extractable/leachable molecules.

WO 2010/124950 A1 and JP 2017-125033 A disclose polymeric thioxanthones.

The use of oligomeric/polymeric photoinitiators is not an ideal solution, as trade-offs typically include reduced cure speed, limited resin compatibility, and increased formulation cost due to reduced active content of polymeric photo initiation systems. In some cases, polymeric photoinitiators will also unfavorably affect cured material properties due to the unoptimized nature of the initiator polymer backbone for any given application. Accordingly, it would be useful to have available monomeric photoinitiators that may be reacted into the backbone during the photoinitiated reaction that forms a polymer.

The inventors have prepared unique photoinitiators having at least two hydroxyl groups that are capable of reaction with other monomers, such as polyisocyanates to form polymers that have the photoinitiator moieties in their backbones. Therefore, photopolymerizable compositions that include these photoinitiators may not require additional photoinitiators. Since the chromophore-containing material is included in the final polymer, there is no danger of the photoinitiators leaching or evaporating out of the final polymer thus contributing to undesirable volatile organic content. Another advantage is that the photopolymerizable composition including these monomers and related oligomers containing these monomers, is that the chromophore/photoinitiator is by definition bound to and compatible with the oligomer and thus the risk of phase separation or altered mechanical properties of the cured material due to an oligomeric photoinitiator component are minimized.

### SUMMARY

The invention relates to a photoinitiator according to the following formula (I): 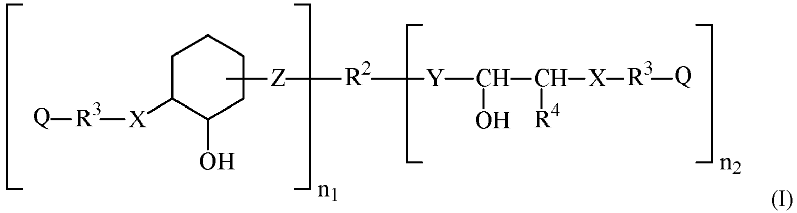 wherein n₁, n₂, Q, X, Y, Z, R¹, R², R³ and R⁴ are as defined herein.

The invention also relates to a method of preparing a photoinitiator by reacting a chromophore-bearing component a) with a polyepoxide component b), wherein component a) comprises at least one compound bearing at least one chromophore moiety and at least one epoxide-reactive group; and component b) comprises at least one compound bearing at least two epoxide rings.

The invention also relates to the use of the photoinitiator according to the invention, as a photoinitiating system in a radiation curable composition.

The invention further relates to the use of the photoinitiator according to the invention, in a photopolymerization reaction.

The invention also relates to use of the photoinitiator according to the invention, to obtain a photoreactive oligomer, in particular a photoreactive urethane oligomer, more particularly a photoreactive urethane (meth)acrylate oligomer.

### DETAILED DESCRIPTION

### Definitions

In the present application, the term "comprise(s) a/an" means "comprise(s) one or more".

Unless mentioned otherwise, the % by weight in a compound or a composition are expressed based on the weight of the compound, respectively of the composition.

Molecular weights herein are number averaged molecular weights measured using gel permeation chromatography with polystyrene standards, unless stated otherwise.

As used herein, the term, "independently selected" with respect to choices of groups in a structure, means that if there are more than one of the group present in the structure, they need not all be the same, as long as they are selected from the listed choices. For example, the recitation, "R³ may be independently selected from a direct bond or a linker" means that one R³ in a structure may be a direct bond and another may be a linker for example.

According to some embodiments, the Rₐ groups in a structure may all be the same. According to some embodiments, the R'ₐ groups in a structure may all be the same. According to some embodiments, the R³ groups in a structure may all be the same. According to some embodiments, the R⁴ groups in a structure may all be the same. According to some embodiments, the X groups in a structure may all be the same. According to some embodiments, the Y groups in a structure may all be the same. According to some embodiments, the Z groups in a structure may all be the same. According to some embodiments, the Q groups in a structure may all be the same.

A "chromophore moiety" means a moiety comprising a group capable of absorbing light and generating a reactive species useful to initiate a polymerisation reaction. In particular, the chromophore moiety may be a Norrish Type II chromophore moiety, i.e. a chromophore moiety that does not fragment upon exposure to radiation and so will not typically initiate radical-chain polymerisation unless a co-initiator such as an amine synergist is present. Upon exposure to radiation, interaction between the Type II chromophore moiety and the co-initiator leads to the generation of radical species which can initiate the polymerisation of UV-curable resins.

"Equivalents" as used herein means the number of moles of a particular reactant that are needed to react with all of the moles of another reactant that may have a different number of reactive groups. For example, when reacting the hydroxyl groups of a monoalcohol with the epoxy groups of a triepoxide, three moles, or three "equivalents" of the monoalcohol are needed to completely react with one mole of the triepoxide.

As used herein, the term "glycidyl ether group" means a group of the following formula (3):

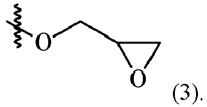

As used herein, the term "glycidyl ester group" means a group of the following formula (4):

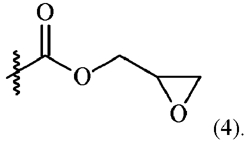

As used herein, the term "glycidyl amine group" means a group of the following formula (5): 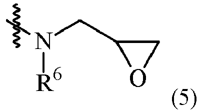 wherein R⁶ is H, alkyl, aryl or a glycidyl group of formula

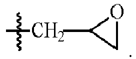

As used herein, the term "glycidyl amide group" means a group of the following formula (6): 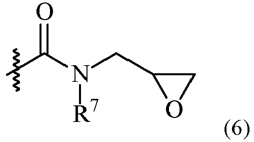 wherein R⁷ is H, alkyl, aryl or a glycidyl group of formula

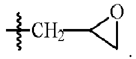

As used herein, the term "cycloaliphatic epoxide group" means a group of the following formula (7):

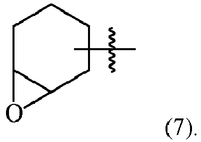

As used herein, the term "epoxy group derived from the epoxidation of a non-cyclic carbon-carbon double bond" means a group of the following formula (8): 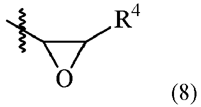 wherein R⁴ is H, an optionally substituted alkyl or an optionally substituted alkenyl, and the epoxide ring is not directly connected to a group of formula ^{∗}-CH₂-O-, ^{∗}-CH₂-O-C(=O)-, ^{∗}-CH₂-NR⁷- or ^{∗}-CH₂-NR⁷-C(=O)-, wherein the symbol * represent the point of attachment to the epoxide ring (i.e. the epoxide ring is not included in a glycidyl ether group, a glycidyl ester group, a glycidyl amine group, a glycidyl amide group, or a cycloaliphatic epoxide).

The term « C1-C6 » refers to the number of carbon atoms comprised in a specific group or linker. For example, a C1-C6 alkylene is an alkylene comprising from 1 to 6 carbon atoms.

The term « alkyl » means a monovalent saturated acyclic hydrocarbon group of formula -CₙH₂ₙ₊₁ wherein n is 1 to 20. An alkyl may be linear or branched. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term « heteroatom-containing alkyl » means an alkyl comprising one or more heteroatoms independently selected from O, N or S.

The term « cycloalkyl » means a monovalent saturated hydrocarbon group comprising a cycle. Examples of cycloalkyl groups include cyclopentyl, cyclohexyl and isobornyl.

The term « heterocycloalkyl » means a cycloalkyl having at least one ring atom that is a heteroatom selected from O, N or S.

The term « alkenyl » means a monovalent acyclic hydrocarbon group comprising at least one carbon-carbon double bond. An alkenyl may be linear or branched.

The term « alkynyl » means a monovalent acyclic hydrocarbon group comprising at least one carbon-carbon triple bond. An alkynyl may be linear or branched.

The term « aryl » means an optionally substituted polyunsaturated aromatic group. The aryl may contain a single ring (i.e. phenyl) or more than one ring wherein at least one ring is aromatic. When the aryl comprises more than one ring, the rings may be fused, linked via a covalent bond (for example biphenyl). The aromatic ring may optionally comprise one to two additional fused rings (i.e. cycloalkyl, heterocycloalkyl or heteroaryl). The term "aryl" also encompasses partially hydrogenated derivatives of the carbocyclic system is described above. Examples include phenyl, naphtyl, biphenyl, phenanthrenyl and naphthacenyl.

The term « heteroaryl » means an aryl having at least one ring atom that is a heteroatom selected from O, N or S.

The term « aralkyl » means an aryl substituted by an alkyl group. An example of an aralkyl group is tolyl.

The term « alkaryl » means an alkyl substituted by an aryl group. An example of an alkaryl group is benzyl (-CH₂-Phenyl).

The term « halogen » means an atom selected from Cl, Br, F and I.

The term « alkoxy » means a group of formula -O-alkyl, wherein the alkyl is as defined above.

The term « aryloxy » means a group of formula -O-aryl, wherein the aryl is as defined above.

The term « thioalkyl » means a group of formula -S-alkyl, wherein the alkyl is as defined above.

The term « thioaryl » means a group of formula -S-aryl, wherein the aryl is as defined above.

The term « aralkyloxy » means a group of formula -O-aralkyl, wherein the aralkyl is as defined above.

The term « alkaryloxy » means a group of formula -O-alkaryloxy, wherein the alkaryl is as defined above.

The term « alkylene » means a plurivalent linker derived from an alkane of formula CₘH₂ₘ₊₂, wherein m is 1 to 50, by removing one hydrogen atom at each point of attachment of the linker. An alkylene may be divalent, trivalent, tetravalent or have even higher valencies.

The term « alkenylene » means a plurivalent aliphatic linker comprising at least one carbon-carbon double bond.

The term « heteroatom-containing alkylene » means an alkylene comprising one or more heteroatoms independently selected from O, N or S.

The term « heteroatom-containing alkenylene » means an alkenylene comprising one or more heteroatoms independently selected from O, N or S.

The term « cycloalkylene » means a plurivalent linker comprising a non-aromatic ring. Examples of cycloalkylene groups include cyclopentylene, cyclohexylene and cyclohexylenedimethylene (i.e -CH₂-Cy-CH₂, wherein Cy is a cyclohexylene).

The term « heterocycloalkylene » means a plurivalent linker comprising a non-aromatic ring having at least one ring atom that is a heteroatom selected from O, N or S.

The term « arylene » means a plurivalent linker comprising at least one aromatic ring.

The term « heteroarylene » means a plurivalent linker comprising an aromatic ring having at least one ring atom that is a heteroatom selected from O, N or S.

The term « alkylamino » means an alkyl substituted by at least one amino group.

The term « alkylthiol » means an alkyl substituted by at least one thiol group.

The term « hydroxyalkyl » means an alkyl substituted by at least one hydroxy group.

The term « haloalkyl » means an alkyl substituted by at least one halogen.

The term « perfluoroalkyl » means an alkyl wherein all of the hydrogen atoms are replaced with fluorine atoms.

The term « linker » means a plurivalent group comprising at least one carbon atom and/or at least one heteroatom such as O, N or S. A linker may connect at least two moieties of a compound together, in particular 2 to 4 moieties of a compound together. For example, a linker that connects two moieties of a compound together is referred to as a divalent linker, a linker that connects three moieties of a compound together is referred to as a trivalent linker, etc....

The term « aliphatic compound, group or linker » means a non-aromatic compound, group or linker. Compounds, groups or linkers comprising a non-aromatic ring (i.e. a cycloaliphatic ring) are encompassed by the term aliphatic compound, group or linker. It may be linear or branched, saturated or unsaturated, cyclic or acyclic. It may be substituted by one or more groups, for example selected from alkyl, hydroxyl, halogen (Br, Cl, I), isocyanate, carbonyl (=O), amine, carboxylic acid, -C(=O)-OR', -C(=O)-O-C(=O)-R', each R' being independently a C1-C6 alkyl. It may comprise one or more bonds selected from ether, ester, amide, urethane, urea, carbonate, organosiloxane, and mixtures thereof.

The term « aromatic compound, group or linker » means a compound, group or linker comprising at least one aromatic ring (i.e. a ring respecting Hückel's aromaticity rule, such as a phenyl), in particular one, two or three, preferably one or two, aromatic rings. Araliphatic compounds, groups or linkers, i.e. comprising both an aromatic moiety and a non-aromatic moiety, are encompassed by the term aromatic compound, group or linker. It may be substituted by one or more groups as defined for the term « aliphatic compound, group or linker ». It may comprise one or more bonds as defined for the term « aliphatic compound, group or linker ».

The term « acyclic compound, group or linker » means a compound, group or linker that does not comprise any rings.

The term « cyclic compound, group or linker » means a compound, group or linker that comprises at least one aromatic or non-aromatic ring.

The term « saturated compound, group or linker » means a compound, group or linker that does not comprise any double or triple carbon-carbon bonds.

The term « unsaturated compound, group or linker » means a compound, group or linker that comprises one or more double or triple carbon-carbon bonds, in particular one or more double carbon-carbon bonds.

The term « polyol » means a compound comprising at least two hydroxyl groups.

As used herein, the term "residue of a polyol" means the moiety that is obtained by removing the hydroxy groups of a polyol.

As used herein, the term "polyacid" means a compound bearing at least two carboxylic acid groups.

As used herein, the term "residue of a polyacid" means the moiety that is obtained by removing the carboxylic acid groups of a polyacid.

As used herein, the term "polyamine" means a compound bearing at least two primary and/or secondary amino groups.

As used herein, the term "residue of a polyamine" means the moiety that is obtained by removing the primary and/or secondary amino groups of a polyamine.

As used herein, the term "hydroxy-acid" means a compound bearing at least one hydroxyl group and at least one carboxylic acid group.

As used herein, the term "residue of a hydroxy-acid" means the moiety that is obtained by removing the hydroxyl and carboxylic acid groups of a hydroxyacid.

As used herein, the term "hydroxy-amine" means a compound bearing at least one hydroxy group and at least one primary and/or secondary amino group.

As used herein, the term "residue of a hydroxy-amine" means the moiety that is obtained by removing the hydroxyl and primary and/or secondary amino groups of a hydroxyamine.

The term « hydroxyl group » means a -OH group.

The term « amino group » means a -NRₐ₁R_{b1} group, wherein Rₐ₁ and R_{b1} are independently H or an optionally substituted alkyl. A « primary amino group » means a -NRₐ₁R_{b1} group, wherein Rₐ₁ and R_{b1} are H. A « secondary amino group » means a -NRₐ₁R_{b1} group, wherein Rₐ₁ is H and R_{b1} is an optionally substituted alkyl.

The term « carboxylic acid group » means a -COOH group.

The term « isocyanate group » means a -N=C=O group.

The term « direct bond » means a covalent bond.

The term « ester bond » means a -C(=O)-O- or -O-C(=O)- bond.

The term « ether bond » means a -O- bond.

The term « organosiloxane bond » means a -Si(R_{c1})₂-O- bond, wherein R_{c1} is an organic group, in particular an organic group selected from alkyl, alkoxy and aryl.

The term « carbonate bond » means a -O-C(=O)-O- bond.

The term « urethane or carbamate bond » means a -NH-C(=O)-O- or -O-C(=O)-NH- bond.

The term « urethane oligomer » means an oligomer comprising at least two urethane bonds.

The term « polyisocyanate » means a compound comprising at least two isocyanate groups.

The term « optionally substituted compound, group or linker » means a compound, group or linker optionally substituted by one or more groups selected from halogen, alkyl, cycloalkyl, heterocycloalkyl, aryl, heteroaryl, alkoxy, aryloxy, aralkyl, alkaryl, aralkyloxy, alkaryloxy, haloalkyl, - OH, -SH, hydroxyalkyl, thioalkyl, thioaryl, alkylthiol, amino, alkylamino, isocyanate, nitrile, oxo (=O), -C(=O)-R', -O-C(=O)-R', -C(=O)-OR', -C(=O)-N(R')₂, -NR'-C(=O)-R', -C(=O)-O-C(=O)-R' and -SO₂-N(R')₂, each R' being independently H or an optionally substituted group selected from alkyl, aryl and alkylaryl.

### Photoinitiator of formula (I)

The photoinitiator of the invention corresponds to the following formula (I): 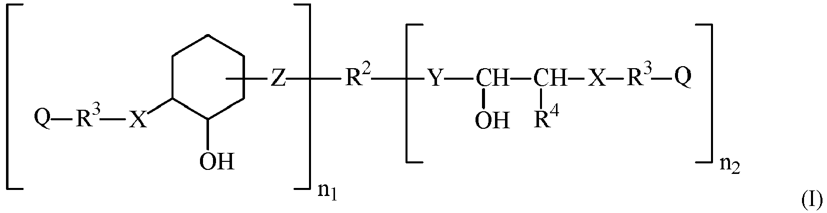 wherein
n₁ is 0, 1, 2, 3 or 4;
n₂ is 0, 1, 2, 3 or 4;
the sum n₁ + n₂ is equal to 1, 2, 3 or 4;
each Q is independently a chromophore moiety;
each X is independently -NR¹-, -O-, -S-, *-C(=O)-O-, or ^{∗}-C(=O)-NR¹-;
each Y is independently a direct bond, #-O-CH₂-, #-C(=O)-O-CH₂-, #-NR⁵-CH₂- or #-C(=O)-NR⁵-CH₂-;
each Z is independently a direct bond, -CH₂-O-C(=O))-# or -C(=O)-O-#;
each R¹ is independently H, alkyl or aryl;
R² is a direct bond or a linker;
each R³ is independently a direct bond or a linker;
each R⁴ is independently H, an optionally substituted alkyl or an optionally substituted alkenyl;
each R⁵ is independently H, alkyl, aryl or -CH₂-CH(OH)-CH₂-X-R³-Q wherein Q, X and R³ are as defined above;
the symbol * represents a point of attachment to R³;
the symbol # represents a point of attachment to R².

In formula (I), the value of n₁ is 0, 1, 2, 3 or 4. In particular, the value of n₁ may be 0, 1 or 2, more particularly n₁ may be 0.

In formula (I), the value of n₂ is 0, 1, 2, 3 or 4. In particular, the value of n₂ may be 2, 3 or 4, more particularly n₂ may be 2 or 3.

In formula (I), the sum n₁ + n₂ is equal to 1, 2, 3 or 4. In particular, the sum n₁ + n₂ may be equal to 2, 3 or 4, more particularly the sum n₁ + n₂ may be equal to 2 or 3. If the sum n₁ + n₂ is equal to 1, then n₁ is preferably 0, n₂ is preferably 1, Y is preferably #-NR⁵-CH₂- or #-C(=O)-NR⁵-CH₂- and R⁵ is preferably -CH₂-CH(OH)-CH₂-X-R³-Q.

In one embodiment, the value of n₁ may be 0 and the value of n₂ may be 1, 2, 3 or 4. In particular, the value of n₁ may be 0, the value of n₂ may be 2 or 3. In another embodiment, the value of n₂ may be 0 and the value of n₁ may be 2, 3 or 4. In particular, the value of n₂ may be 0, the value of n₁ may be 2. In another embodiment, the value of n₁ may be 1, 2 or 3 and the value of n₂ may be 1, 2 or 3. In particular, the value of n₁ may be 1 and the value of n₂ may be 1 or 2.

In a preferred embodiment, n₂ is other than 0 and R² bears at least one moiety corresponding to the following formula (1): 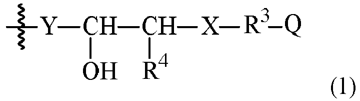
wherein Q, X, Y, R³ and R⁴ are as defined herein.
R² may bear at least one moiety of formula (1) wherein Y is #-O-CH₂- and R⁴ is H. Such a moiety may be derived from the ring-opening of a glycidyl ether group.
R² may bear at least one moiety of formula (1) wherein Y is #-(C=O)-O-CH₂- and R⁴ is H. Such a moiety may be derived from the ring-opening of a glycidyl ester group.
R² may bear at least one moiety of formula (1) wherein Y is #-NR⁵-CH₂-, R⁴ is H and R⁵ is as defined above. Such a moiety may be derived from the ring-opening of a glycidyl amine group.
R² may bear at least one moiety of formula (1) wherein Y is #-(C=O)-NR⁵-CH₂-, R⁴ is H and R⁵ is as defined above. Such a moiety may be derived from the ring-opening of a glycidyl amide group.
R² may bear at least one moiety of formula (1) wherein Y is a direct bond and R⁴ is H, an optionally substituted alkyl or an optionally substituted alkenyl. Such a moiety may be derived from the ring-opening of an epoxy group which is derived from the epoxidation of a non-cyclic carbon-carbon double bond.
R² may bear 1, 2, 3 or 4 or four moieties of formula (1). Such moieties may be identical or different from one another. For example, R² may bear 2, 3 or 4 moieties of formula (1) wherein Y is #-O-CH₂- and R⁴ is H. Alternatively, R² may bear 2, 3 or 4 moieties of formula (1) wherein Y is #-(C=O)-O-CH₂- and R⁴ is H. Alternatively, R² may bear 1 or 2 moieties of formula (1) wherein Y is #-NR⁵-CH₂- and R⁴ is H. Alternatively, R² may bear 1 or 2 moieties of formula (1) wherein Y is #-(C=O)-NR⁵-CH₂- and R⁴ is H. Alternatively, R² may bear two or three moieties of formula (1) wherein Y is a direct bond and R⁴ is H or an optionally substituted alkyl. Alternatively, R² may bear at least two moieties of formula (1), wherein the moieties differ from one another due to the nature of Y and/or R⁴.

In another embodiment, n₁ is other than 0 and R² bears at least one moiety according to the following formula (2): 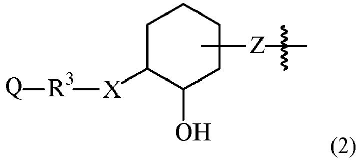 wherein Q, X, Z and R³ are as defined herein.

Such a moiety may be derived from the ring-opening of a cycloaliphatic epoxide group.

R² may bear at least one moiety of formula (2) wherein Z is -CH₂-O-C(=O))-#.

R² may bear at least one moiety of formula (2) wherein Z is -C(=O)-O-#.

R² may bear at least one moiety of formula (2) wherein Z is a direct bond.

R² may bear 2, 3 or 4 or four moieties of formula (2). Such moieties may be identical or different from one another. For example, R² may bear 2, 3 or 4 moieties of formula (2) wherein Z is -CH₂-O-C(=O))-#. Alternatively, R² may bear 2, 3 or 4 moieties of formula (2) wherein Z is -C(=O)-O-#. Alternatively, R² may bear at least two moieties of formula (2), wherein the moieties differ from one another due to the nature of Z.

In yet another embodiment, n₁ and n₂ are both other than 0 and R² bears at least one moiety according to formula (1) and at least one moiety according to formula (2). In particular, R² may bear 1 moiety according to formula (1) wherein Y is a direct bond and R⁴ is H and 1 moiety of formula (2) wherein Z is a direct bond. Alternatively, R² may bear 1 moiety according to formula (1) wherein Y is #-O-CH₂- and R⁴ is H and one moiety of formula (2) wherein Z is a direct bond. Alternatively, R² may bear 2 moieties according to formula (1) wherein Y is #-(C=O)-O-CH₂- and R⁴ is H and 1 moiety of formula (2) wherein Z is a direct bond.

The photoinitiator of formula (I) may correspond to one of the following formulae (I-a) to (I-n): 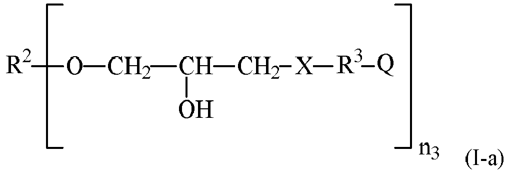 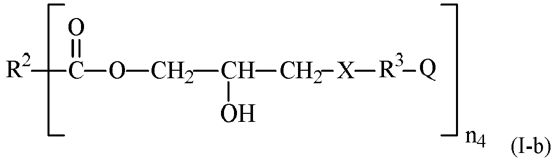 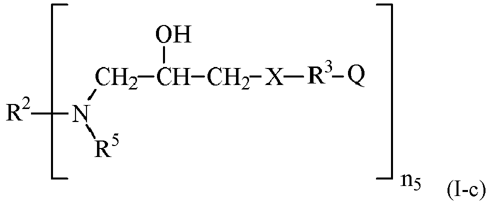 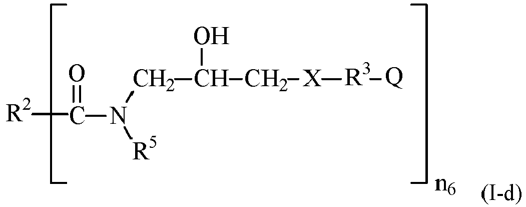 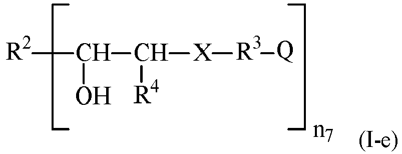 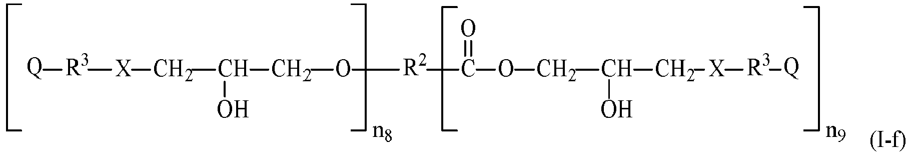 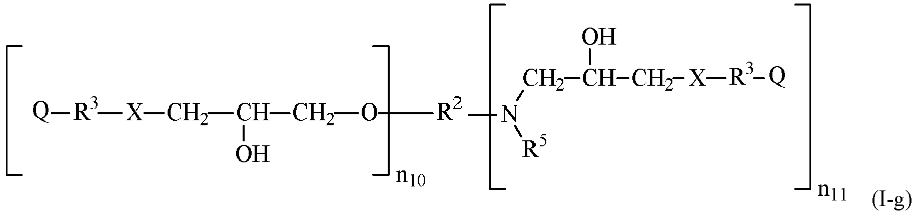 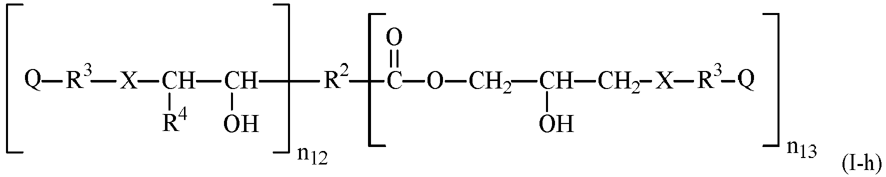 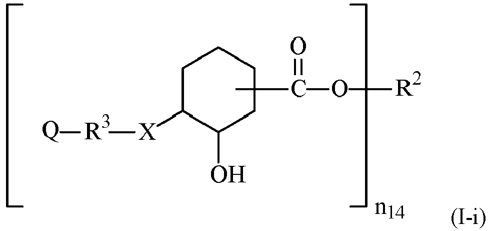 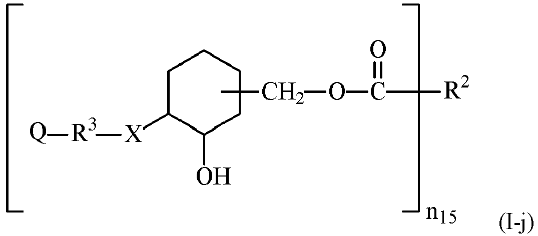 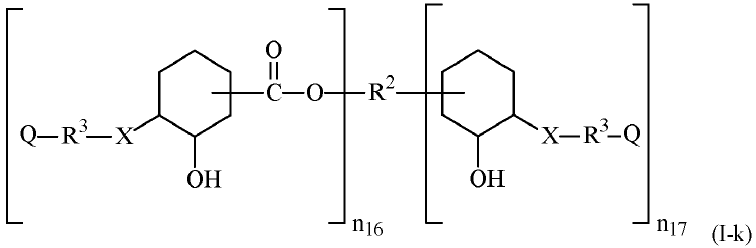 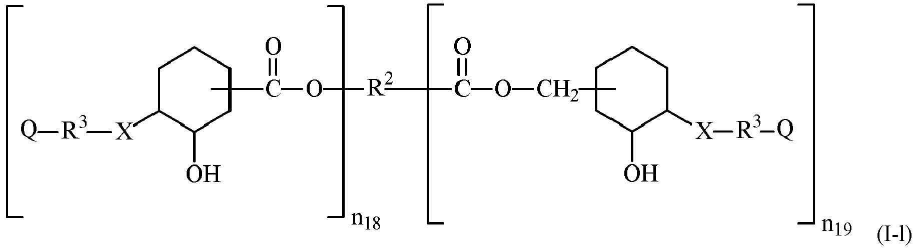 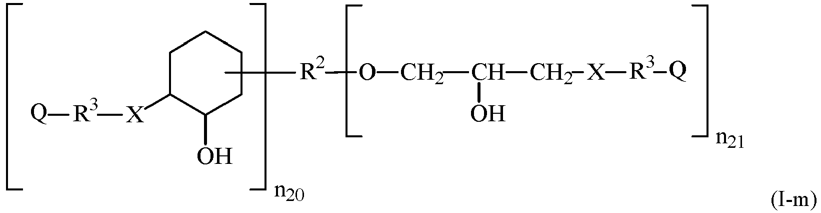 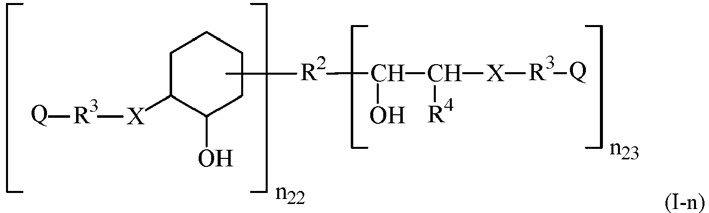 wherein
Q, X, R², R³, R⁴ and R⁵ are as defined herein;
n₃, n₄, n₇, n₁₄ and n₁₅ are independently 2, 3 or 4, preferably 2 or 3;
n₅ and n₆ are independently 1 or 2, preferably 2;
n₈ is 1, 2, or 3 preferably 2;
n₉ is 1, 2 or 3, preferably 1;
n₁₀, n₁₁, n₁₆, n₁₇, n₁₈, n₁₉, n₂₀, n₂₁, n₂₂ and n₂₃ are independently 1 or 2, preferably 1;
n₁₂ and n₁₃ are independently 1, 2 or 3, preferably 1.

Photoinitiators of formula (I-a), (I-b), (I-c), (I-e), (I-f), (I-g) and (I-h) are preferred. Photoinitiators of formula (I-a), (I-b), (I-c) and (I-e) are particularly preferred.

The photoinitiators of formula (I) and (I-a) to (I-n) comprise at least one, preferably at least two, chromophore moieties Q. In particular, the photoinitiators of formula (I) and (I-a) to (I-n) comprise 2, 3 or 4 chromophore moieties Q.

Each chromophore moiety Q may independently comprise a benzophenone moiety, a thioxanthone moiety, an anthraquinone moiety, a xanthone moiety or an acridone moiety.

In particular, each chromophore moiety Q may independently be a benzophenone moiety according to the following formula (A) or a thioxanthone, anthraquinone, xanthone or acridone moiety according to the following formula (B): 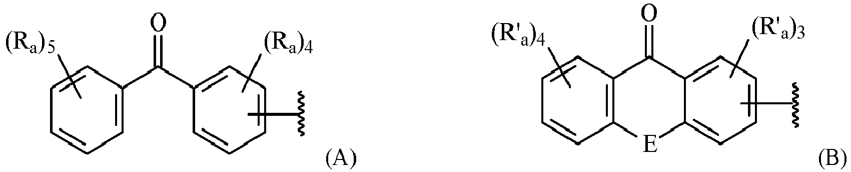 wherein
E is S, O, C(=O) or NR, in particular S;
R is H, an optionally substituted alkyl or an optionally substituted aryl;
each Rₐ and R'ₐ is independently H, F, Cl, Br, I, -OR_{b}, -SR_{b}, -N(R_{b})₂, -NOz, -CN, -C(=O)R_{b}, -O-C(=O)R_{b}, -C(=O)OR_{b}, -C(=O)N(R_{b})₂, -NR_{b}-C(=O)-R_{b}, -SO₂-N(R_{b})₂ or an optionally substituted group selected from alkyl, heteroatom-containing alkyl, cycloalkyl, heterocycloalkyl, alkenyl, alkynyl, aryl, aralkyl, alkaryl and heteroaryl;
two adjacent Rₐ or R'ₐ groups may form, with the carbon atoms to which they are attached, a 5 to 8 membered ring;
each R_{b} is independently H or an optionally substituted group selected from alkyl, heteroatom-containing alkyl, cycloalkyl, heterocycloalkyl, alkenyl, alkynyl, aryl, aralkyl, alkaryl and heteroaryl.

More particularly, each chromophore moiety Q may independently be a benzophenone moiety according to the following formula (A1) or a thioxanthone moiety according to the following formula (B1): 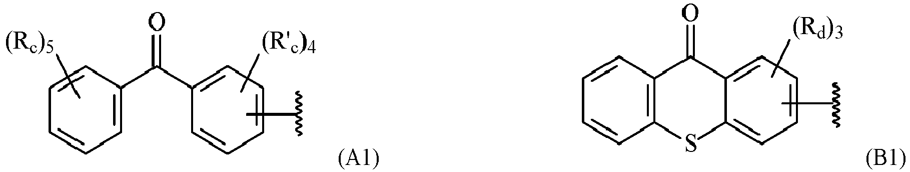 wherein
each R_{c} is independently H, alkyl, Ar, -S-Ar or -O-Ar wherein Ar is aryl, in particular H, methyl, Ph, -S-Ph or -O-Ph wherein Ph is phenyl;
each R'_{c} is independently H or alkyl, in particular H or methyl;
each R_{d} is independently H, halogen, alkoxy, alkyl or -C(=O)-Ar wherein Ar is aryl, in particular H, F, Cl, methyl, ethyl, isopropyl or -C(=O)-Ph wherein Ph is phenyl.

In a preferred embodiment, all of the Q moieties have the same structure. In particular, each chromophore moiety Q may be a benzophenone moiety according to formula (A1). Alternatively, each chromophore moiety Q may be a thioxanthone moiety according to formula (B1).

In the photoinitiators of formula (I) and (I-a) to (I-n), each chromophore moiety Q is connected to a R³.

Each R³ is independently a direct bond or a linker.

When Q is according to formula (A), then R³ is preferably a direct bond.

When Q is according to formula (B), then R³ is preferably a linker.

When Q is according to formula (B), E is S and R³ is a direct bond, then X is preferably not -O-.

When R³ is a direct bond, then X is preferably -NR¹-, *-C(=O)-O- or ^{∗}-C(=O)-NR¹-.

When R³ is a linker, said linker may be a divalent linker comprising 1 to 15, preferably 1 to 10, carbon atoms. Said linker may further optionally comprise one or more heteroatoms, such as O, N or S. For example, the linker may be a hydrocarbon linker optionally comprising one or more bonds selected from -O-, -S-, -NR₁-, -C(=O)-, -O-C(=O)-, -C(=O)-O-, -NR₁-C(=O)-, -C(=O)-NR₁-, -O-C(=O)-O-, -NR₁-C(=O)-O-, -O-C(=O)-NR₁-, and combinations thereof, wherein R₁ is H, alkyl or aryl.

When R³ is a linker, said linker may be:
- an acyclic linker which may be saturated or unsaturated, in particular an acyclic linker having from 1 to 6, from 1 to 4 or from 1 to 2 carbon atoms; or
- a cyclic linker which may be aromatic or non-aromatic, monocyclic or polycyclic, in particular a cyclic linker having from 6 to 10, from 7 to 9 or from 7 to 8 carbon atoms.

In a preferred embodiment, each R³ is independently a direct bond, a C1-C6 alkylene, a C1-C6 oxyalkylene, a C1-C6 alkenylene, a C1-C6 thioalkylene, a C1-C6 ketoalkylene or a C1-C6 aminoalkylene.

More particularly, each R³ may independently be a direct bond, an alkylene of formula (C-1), an oxyalkylene of formula (C-2), a thioalkylene of formula (C-3), a ketoalkylene of formula (C-4) or an aminoalkylene of formula (C-5):

•-(CRₘR'ₘ)_{f}-§ (C-1)

•-O-(CRₒR'ₒ)_{g}-§ (C-2)

•-S-(CRₚR'ₚ)ₕ-§ (C-3)

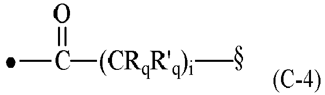

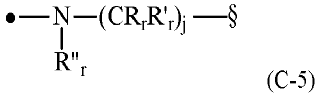

wherein
each Rₘ, R'ₘ, Rₒ, R'ₒ, Rₚ, R'ₚ, R_{q}, R'_{q}, Rᵣ and R'ᵣ is independently H or an optionally substituted alkyl, in particular H;
R"ᵣ is H or an optionally substituted alkyl;
f is 1, 2, 3, 4, 5 or 6, in particular 1 or 2;
g is 1, 2, 3, 4, 5 or 6, in particular 1 or 2;
h is 1, 2, 3, 4, 5 or 6, in particular 1 or 2;
i is 1, 2, 3, 4 or 5, in particular 1;
j is 1, 2, 3, 4, 5 or 6, in particular 1 or 2;
the symbol ● represents a point of attachment to the Q moiety;
the symbol § represents a point of attachment to the X moiety.

More particularly, each R³ may independently be selected from a direct bond, an alkylene of formula (C-1), an oxyalkylene of formula (C-2) and an aminoalkylene of formula (C-5). Even more particularly, each R³ may independently be selected from a direct bond, an alkylene of formula (C-1) and an oxyalkylene of formula (C-2) as defined above.

In the photoinitiators of formula (I) and (I-a) to (I-n), each R³ is connected to X. X may correspond to the residue of an epoxide-reactive group, i.e. a group that is capable of opening an epoxide ring. Examples of epoxide reactive groups are alcohols, thiols, primary and secondary amines, carboxylic acids and amides.

Each X is independently -NR¹-, -O-, -S-, *-C(=O)-O-, or ^{∗}-C(=O)-NR¹-; each R¹ is independently H, alkyl or aryl and the symbol * represents a point of attachment to R³.

In particular, each X may independently be -NR¹-, -O-, or *-C(=O)-O-. More particularly, each X may independently be -NR¹- or *-C(=O)-O-.

In the photoinitiators of formula (I) and (I-a) to (I-n), each R² is independently a direct bond or a linker. When R² is a linker, the linker may be divalent, trivalent or tetravalent. When R² is a linker, the linker may be aliphatic or aromatic, in particular aliphatic.

In a preferred embodiment, R² is a direct bond or a linker other than a polyether linker. As used herein, the term "linker other than a polyether linker" means a linker that does not comprise consecutive repeating units derived from the ring opening of a cyclic ether comprising 2 to 4 carbon atoms. In particular, a linker other than a polyether may be devoid of a moiety containing more than one oxyalkylene units, preferably a linker other than a polyether does not comprise any oxyalkylene units. More particularly, a linker other than a polyether may be devoid of the following moiety -[O-A¹]_{z}-wherein A¹ is a C2-C4 alkylene and z is more than 1, preferably a linker other than a polyether does not comprise any -[O-A']- moiety wherein A¹ is a C2-C4 alkylene. Even more particularly, a linker other than a polyether may not comprise more than one non-cyclic ether bonds, preferably, a linker other than a polyether does not comprise any non-cyclic ether bonds.

In a particularly preferred embodiment, R² is a direct bond or a non-polymeric linker. As used herein, the term "non-polymeric linker" means a linker that does not comprise consecutive repeating units other than consecutive carbon atoms.

R² may comprise 0 to 20, in particular 0 to 15, more particularly 0 to 10, carbon atoms. R² may comprise 0 to 2, in particular 0 to 1, more particularly 0, oxygen atoms. R² may comprise 0 to 2, in particular 0 to 1, more particularly 0, heteroatoms atoms selected from O, N or S.

The molecular weight of R² may be lower than 500 g/mol, in particular lower than 400 g/mol, more particularly lower than 300 g/mol, even more particularly lower than 250 g/mol, more particularly still lower than 200 g/mol.

In particular, each R² may independently be a direct bond or a linker selected from the group consisting of alkylene, heteroatom-containing alkylene, alkenylene, heteroatom-containing alkenylene, cycloalkylene, heterocycloalkylene, arylene, heteroarylene, and combinations thereof. In particular, R² may be a direct bond or a linker selected from the group consisting of alkylene, heteroatom-containing alkylene, cycloalkylene, arylene and combinations thereof.

In one embodiment, R² may be an aromatic linker such as arylene, heteroarylene or combinations thereof. In such a case, R² is preferably arylene.

For example, R² may be represented by one of the following formulae (3) to (9): 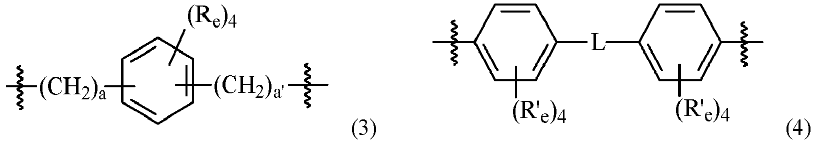 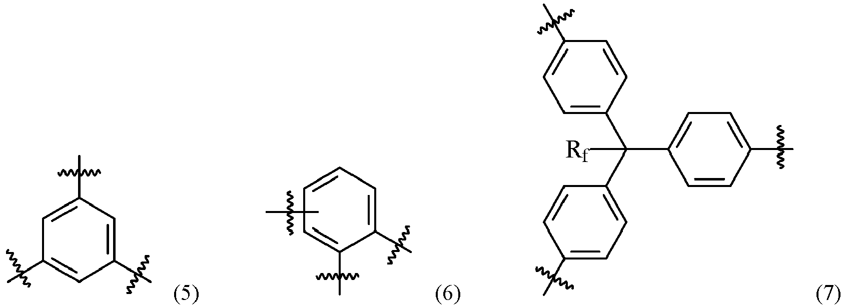 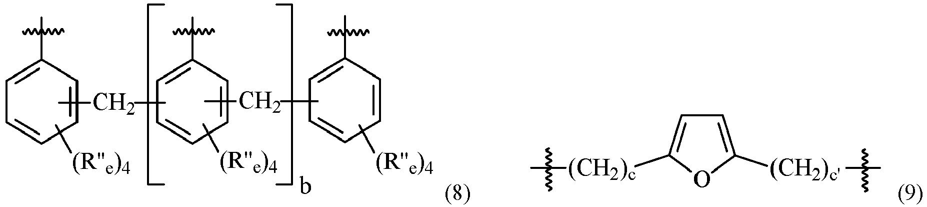
wherein L is a direct bond or a linker;
Rₑ, R'ₑ and R"ₑ are independently selected from H, alkyl, cycloalkyl, aryl, alkaryl, aralkyl, alkoxy, - C(=O)O-Alkyl and a halogen atom;
R_{f} is H or methyl;
a, a', c and c' are independently 0 or 1;
b is 1 or 2.

In particular, R² may be represented by one of formulae (3) or (4) as defined above, preferably by formula (4). When R² is represented by formula (4), L may be selected from direct bond, alkylene, -CR₃R₄-, -C(=O)-, -C(=O)-O-Alk-O-C(=O)-, -SO-, -SOz-, -C(=CCl₂)- and -CR₅R₆-Ph-CR₇R₈-; wherein
R₃ and R₄ are independently selected from H, alkyl, cycloalkyl, aryl, haloalkyl and perfluoroalkyl, or
R₃ and R₄, together with the carbon atoms to which they are attached, may form a ring;
R₅, R₆, R₇ and R₈ are independently selected from H, alkyl, cycloalkyl, aryl, haloalkyl and perfluoroalkyl;
Alk is an alkylene;
Ph is a phenylene optionally substituted with one or more groups selected from alkyl, cycloalkyl, aryl and a halogen atom.

More particularly, R² may correspond to the residue of an optionally substituted aromatic polyol, polyacid, polyamine, hydroxy-acid or hydroxy-amine (without the OH, COOH and/or amino groups).

For example, R² may be the residue of an optionally substituted aromatic polyol, polyacid, polyamine, hydroxy-acid or hydroxy-amine comprising 1 to 3, preferably 1 or 2, aromatic rings, such as pyrocatechol, resorcinol, cardol, (hydroxymethyl)phenol, benzenedimethanol, a hydroxybenzoic acid, phthalic acid, terephthalic acid, isophthalic acid, naphthalene dicarboxylic acid, a bisphenol, a biphenyl diol, phloroglucinol, pyrogallol, tris(hydroxyphenyl)methane, tris(hydroxyphenyl)ethane, trimellitic acid, gallic acid, a condensation product of an optionally substituted aromatic alcohol and formaldehyde (also referred to as novolak), phenylene diamine, toluylene diamine, xylylene diamine, diaminobiphenyl, diaminodiphenyl ether, diaminodiphenyl methane, diaminodiphenyl sulfone, aminophenol, (aminophenoxy)phenol.

Even more particularly, R² may be the residue of an optionally substituted bisphenol. Examples of suitable bisphenols are bisphenol A, bisphenol AP, bisphenol AF, bisphenol B, bisphenol BP, bisphenol C, bisphenol C2, bisphenol F, bisphenol G, bisphenol M, bisphenol S, bisphenol P, bisphenol PH, bisphenol TMC, bisphenol-Z, dinitrobisphenol A, tetrabromobisphenol A and combinations thereof.

In another embodiment, R² may be an aliphatic linker, such as alkylene, heteroatom-containing alkylene, cycloalkylene, heterocycloalkylene, or combinations thereof. In such a case, R² is preferably alkylene, heteroatom-containing alkylene, cycloalkylene or combinations thereof.

More particularly, R² may be represented by one of the following formulae (10) to (20): 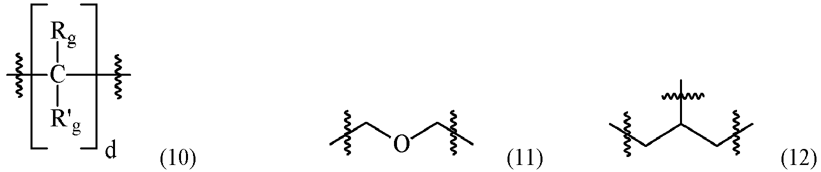 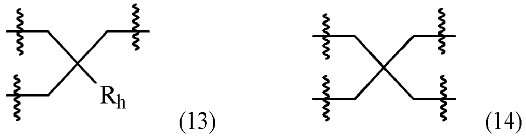 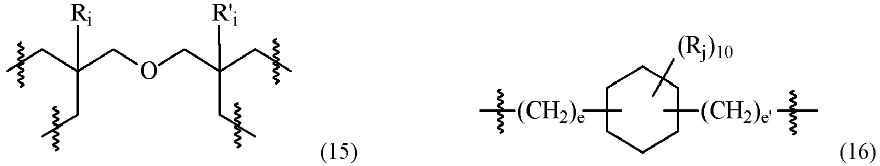 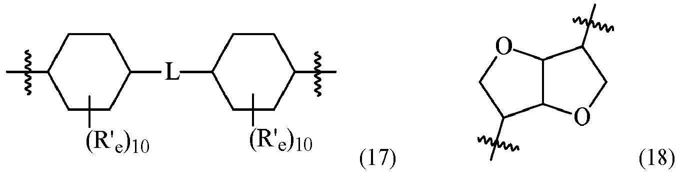 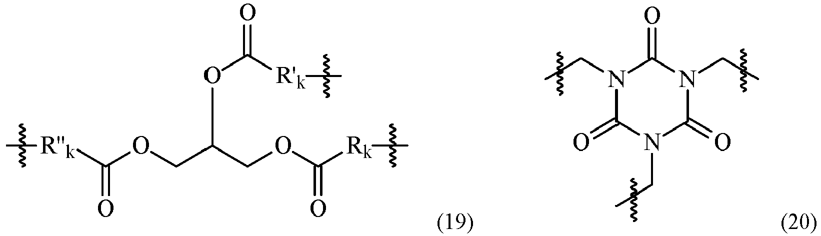 wherein
R'ₑ and L are as defined above for formula (4);
each R_{g}, R'_{g}, Rₕ, Rᵢ and R'ᵢ is independently H or alkyl;
each Rⱼ is independently H, alkyl, cycloalkyl, aryl, alkaryl, aralkyl, alkoxy, -C(=O)O-Alkyl and a halogen atom;
Rₖ, R'ₖ and R"ₖ are independently alkylene or alkenylene;
d is 1 to 12;
e and e' are independently 0 or 1.

In particular, R² may be represented by one of formulae (10), (11), (12), (13), (15), (16), (17) or (19) as defined above, preferably by one of formulae (10), (11) or (12).

R² may correspond to the residue of an optionally substituted aliphatic polyol, polyacid, polyamine, hydroxy-acid or hydroxy-amine (without the OH, COOH and/or amino groups).

For example, R² may be the residue of an optionally substituted aliphatic polyol, polyacid, polyamine, hydroxy-acid or hydroxy-amine selected from ethylene glycol, 1,2- or 1,3-propylene glycol, 1,2-, 1,3- or 1,4-butylene glycol, 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, neopentyl glycol, 2,2-diethyl-1,3-propanediol, 3-methyl-1,5-pentanediol, 3,3-dimethyl-1,5-pentanediol, 2,4-diethyl-1,5-pentanediol, 3,3-butylethyl-1,5-pentane diol, cyclohexanediol, cyclohexane-1,4-dimethanol, norbornene dimethanol, norbornane dimethanol, tricyclodecanediol, tricyclodecane dimethanol, hydrogenated bisphenol A, B, F or S, trimethylolmethane, trimethylolethane, trimethylolpropane, di(trimethylolpropane), pentaerythritol, glycerol, a dianhydrohexitol (i.e. isosorbide, isomannide, isoidide), a hydroxylated vegetable oil, tris(2-hydroxyethyl)isocyanurate, 1,2-ethylenediamine, 1,3-propylenediamine, 1,4-tetramethylenediamine, 1,5-pentamethylenediamine, 1,6-hexamethylenediamine, 1,8-octamethylenediamine, 1,12-dodecamethylenediamine, isophoronediamine, diaminocyclohexane, methylcyclohexane diamine, bis(aminomethyl)cyclohexane, diaminodecahydronaphthalene, dimethyldiaminodicyclohexylmethane, diaminodicyclohexylmethane, bis(aminomethyl)norbomane, malonic acid, succinic acid, 2-methylsuccinic acid, 2,2-dimethylsuccinic acid, glutaric acid, 3,3-diethylglutaric acid, adipic acid, pimelic acid, suberic acid, azelaic acid, sebacic acid, dodecanedioic acid, citric acid, 1,2-, 1,3- or 1,4 cyclohexane dicarboxylic acid, glycolic acid, 12-hydroxystearic acid, ethanolamine, diethanolamine.

Alternatively, R² may correspond to the residue of an epoxidized polyunsaturated compound, i.e. a compound having at least two non-aromatic carbon-carbon double bonds which have been epoxidized.

As used herein, the term "residue of epoxidized polyunsaturated compound" means the residue that is obtained by removing the terminal groups containing an epoxide ring from the compound. For example, if the epoxidized polyunsaturated compound has the following formula: 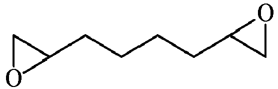 then the residue of the epoxidized polyunsaturated compound corresponds to the following formula:

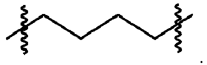

Alternatively, R² may correspond to the residue of an epoxidized oil. As used herein, the term "residue of an epoxidized oil" means the residue that is obtained by removing the terminal groups containing an epoxide from the oil. For example, if the epoxidized oil has the following formula: 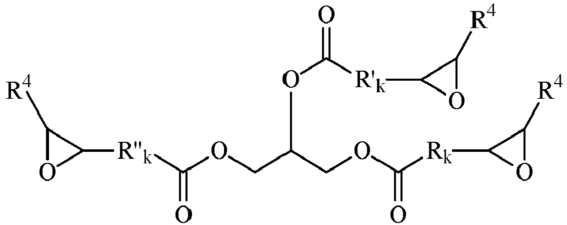 wherein
each R⁴ is independently H, an optionally substituted alkyl or an optionally substituted alkenyl;
Rₖ, R'ₖ and R"ₖ are independently alkylene or alkenylene;
then the residue of the epoxidized oil corresponds to the following formula:

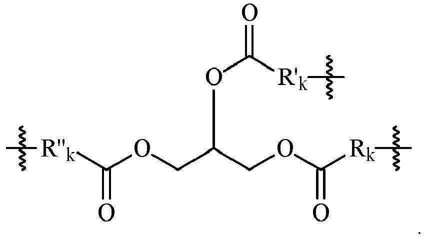

When n₁ is 0, R² is preferably selected from one of formula (3) to (19).

When n₂ is 0, R² is preferably a direct bond or alkylene.

When n₁ and n₂ are both other than 0, R² is preferably a direct bond or alkylene.

In the photoinitiators of formula (I) and (I-a) to (I-n), each R⁴ is independently H, an optionally substituted alkyl or an optionally substituted alkenyl. In one embodiment, each R⁴ is H. In another embodiment, each R⁴ is an optionally substituted alkyl or an optionally substituted alkenyl. In yet another embodiment, part of the R⁴ groups are H and the other part of the R⁴ groups are an optionally substituted alkyl or an optionally substituted alkenyl. Preferably, each R⁴ is H.

When R² bears a moiety of formula (1) wherein R⁴ is an optionally substituted alkyl, then Y is preferably a direct bond.

In the photoinitiators of formula (I), (I-c), (I-d) and (I-g), each R⁵ is independently H, alkyl, aryl or -CH₂-CH(OH)-CH₂-X-R³-Q. In particular, each R⁵ is independently H, alkyl or -CH₂-CH(OH)-CH₂-X-R³-Q.

In a particularly preferred embodiment, the photoinitiator of formula (I) is according to one of the following formulae (I-a), (I-b) (I-c) or (I-e), preferably one of formula (I-a) or (I-e): 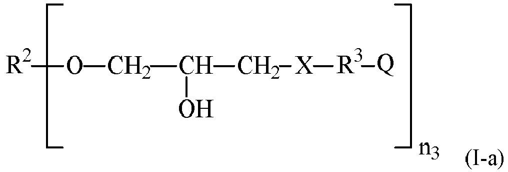 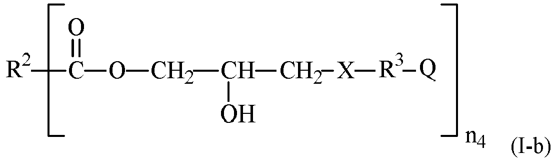 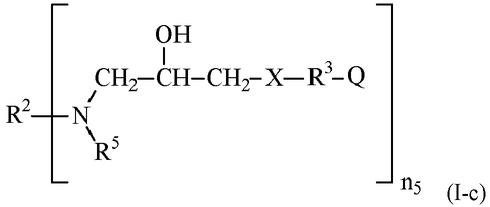 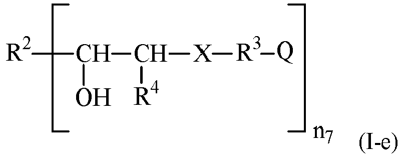 wherein
n₃, n₄, n₅, and n₇ are independently 2, 3 or 4, preferably 2 or 3;
each chromophore moiety Q is according to formula (A) or (B) as defined above, preferably each chromophore moiety Q is according to formula (A1) or (B1) as defined above;
each X is independently -NR¹-, -O- or *-C(=O)-O-, preferably -NR¹- or *-C(=O)-O-;
each R¹ is independently H or alkyl;
each R² is independently an alkylene or a heteroatom-containing alkylene;
each R³ is independently a direct bond, an alkylene of formula (C-1) as defined above, an oxyalkylene of formula (C-2) as defined above or an aminoalkylene of formula (C-5) as defined above, preferably a direct bond, an alkylene of formula (C-1) or an oxyalkylene of formula (C-2);
each R⁴ is H;
each R⁵ is independently H, alkyl or -CH₂-CH(OH)-CH₂-X-R³-Q wherein X, R³ and Q are as defined above;
the symbol * represents a point of attachment to R³.

In a particularly preferred embodiment, the photoinitiator of formula (I) is according to one of the following formulae (I-1) to (I-9):

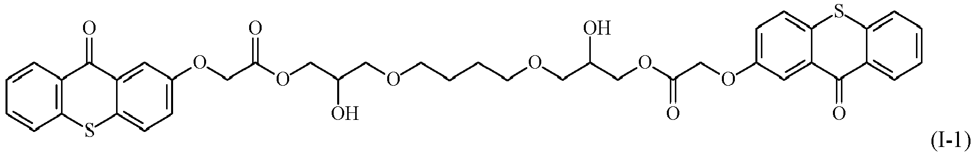

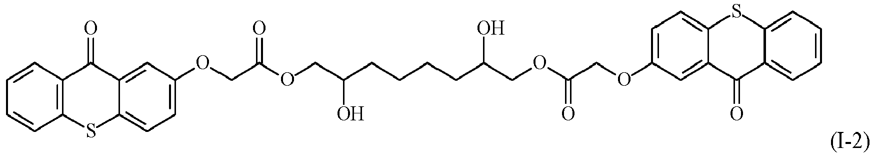

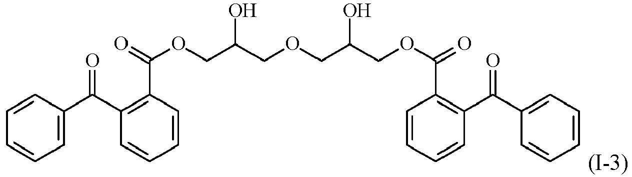

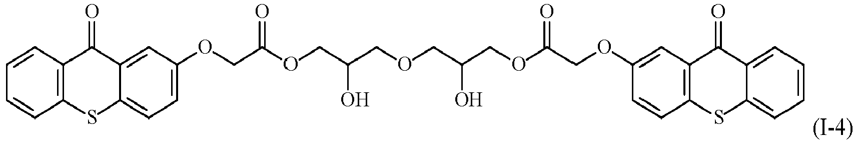

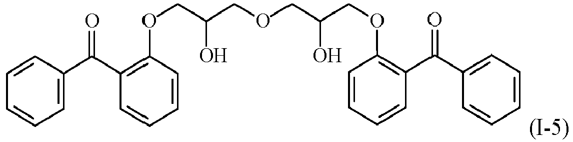

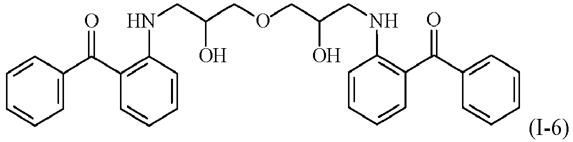

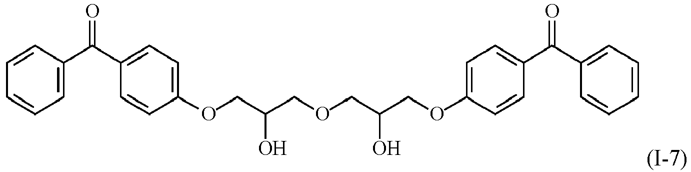

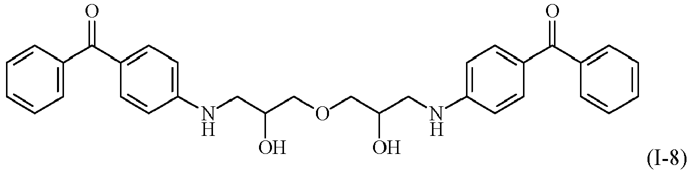

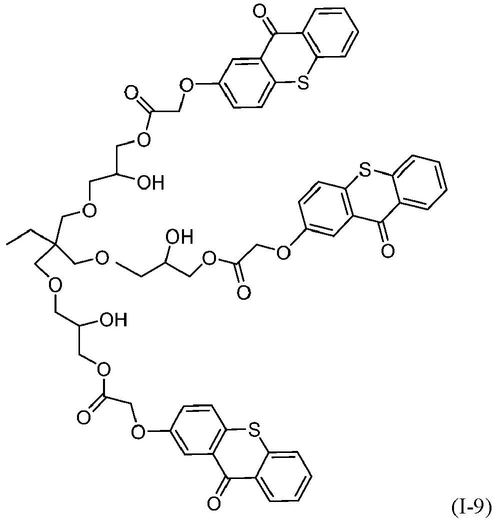

### Method for obtaining a photoinitiator

The invention also relates to a method of preparing a photoinitiator. The method may be used to prepare the photoinitiator according to the invention. The method comprises a step of reacting a polyepoxide component with a chromophore-bearing component that has at least one epoxide-reactive group, i.e. a group that is capable of opening an epoxide ring. The opening of the epoxide rings of the polyepoxide component thus forms free hydroxyl groups that may be useful for further reactions, for example using the photoinitiator to obtain a photoreactive urethane oligomer, a photoreactive polyester oligomer or a polymerizable photoinitiator as detailed below.

The photoinitiator of the invention may be obtained using n equivalents of chromophore-bearing component per equivalent of polyepoxide component. As used herein, n is the number of moles of epoxide rings present in the polyepoxide component. The value for n may be obtained by multiplying the number of moles of polyepoxide component by its epoxide functionality (i.e. the number of epoxide rings on the polyepoxide compound). For example, if the photoinitiator of the invention is obtained using 1 mole of a polyepoxide compound bearing two epoxide rings, then n is equal to 2. If the polyepoxide component comprises a mixture of polyepoxide compounds, then n corresponds to the total number of moles of epoxide rings in the mixture.

The reaction between the polyepoxide component and the chromophore-bearing component may be carried out in the presence of at least one catalyst. The catalyst may be any substance which is capable of catalyzing the ring-opening an epoxide, especially when the epoxide-reactive group of the chromophore-bearing component is a carboxylic acid, an alcohol or a thiol. In particular, the catalyst may be selected from:
- a metal halide, such as iron chloride, aluminum chloride or boron trifluoride;
- a quaternary ammonium salt, such as benzyltriethylammonium chloride, tetrapropylammonium chloride or tetrabutylammonium bromide;
- a mineral acid such as sulfuric acid,
- an organic acid such as acetic acid, trifluoromethanesulfonic anhydride ((CF₃SO₂)₂O, Tf₂O), methanesulfonic anhydride ((CH₃SO₂)₂O), trifluoroacetic anhydride ((CF₃CO)₂O), acetic anhydride ((CH₃CO)₂O)
- an organometallic compound such as tin catalysts (for example dibutyltin dilaurate), carboxylate complexes of bismuth (for example bismuth octoate or bismuth neodecanoate); acetylacetonate complexes of zirconium; acetylacetonate complexes of hafnium; acetylacetonate complexes of titanium; beta-diketiminate complexes of zirconium; beta-diketiminate complexes of hafnium; beta-diketiminate complexes of titanium; amidinate complexes of zirconium; amidinate complexes of hafnium; amidinate complexes of titanium; carboxylate complexes of zinc;
- a quaternary phosphonium salt such as ethyltriphenylphosphonium bromide or tetrapropylphosphonium chloride,
- a tertiary amine such as 2-phenylimidazoline,
- a tertiary phosphine such as triphenylphosphine;
and combinations thereof.

The amount of catalyst may be lower than 5%, lower than 2%, lower than 1% or even lower than 0.5% by weight based on the total weight of the polyepoxide component and chromophore-bearing component.

The reaction between the polyepoxide component and the chromophore-bearing component may be carried out in the presence of a stabilizer, in particular a stabilizer selected from hydroquinone (HQ), hydro-quinone monomethyl ether (MEHQ, 4-methoxyphenol), 4-tert-butylcatechol (TBC), and 3,5-di-tertiobutyl-4-hydroxytoluene (BHT), phenothiazine (PTZ) and mixtures thereof.

The reaction between the polyepoxide component and the chromophore-bearing component may be carried out in the presence of a solvent. Alternatively, the process may not require the presence of a solvent. As used herein, the term "solvent" means a non-reactive organic solvent, i.e. a solvent that does not react with the other components used to prepare the self-crosslinkable urethane (meth)acrylate.

Examples of suitable solvents include aliphatic hydrocarbons such n-pentane, n-hexane, n-heptane, octane cyclohexane or methylcyclohexane; aromatic hydrocarbons such as benzene toluene or xylene; halogenated hydrocarbons such as dichloromethane, chloroform or trichlororethane; ketones such as acetone, methyl ethyl ketone, methylpropylketone, diethylketone, methylisobutylketone, ethylbutylketone, cyclopentanone or cyclohexanone; esters such as methyl formate, butyl formate, methyl acetate, ethyl acetate, propyl acetate or butyl acetate; ethers such as diethyl ether, diisopropyl ether, dibutyl ether, ethylene glycol diethyl ether, tetrahydrofuran or tetrahydropyrane; carbonates such as diethylcarbonate; and combinations thereof.

The reaction between the polyepoxide component and the chromophore-bearing component may be carried out at a temperature of from 50 to 150°C, in particular 70 to 130°C.

The reaction between the polyepoxide component and the chromophore-bearing component may be carried out until the epoxide-reactive group value (ERV) and/or the epoxy value (EV) is lower than 10 mg KOH/g, in particular lower than 5 mg KOH/g, more particularly lower than 2 mg KOH/g. The AV and/or EV may be adjusted by adding more polyepoxide component or chromophore-bearing component as the reaction proceeds as needed.

The reaction between the polyepoxide component and the chromophore-bearing component may be carried out for a time of 1 to 72h, in particular 2 to 24h.

Once the reaction is finished, the reaction medium may be washed one or more times with an aqueous solution, for example an aqueous solution of sodium chloride. The solvent(s) may be evaporated from the resulting organic phase.

### Chromophore-bearing component a)

The photoinitiator of the invention may be derived from the reaction of one or more chromophore-bearing compounds. The one or more chromophore-bearing compounds used to obtain the photoinitiator are herein referred to as component a).

As used herein, the term "chromophore-bearing compound" means a compound bearing at least one chromophore moiety as defined above. In particular, said chromophore moiety may correspond to chromophore moiety Q as defined above for the photoinitiator of the invention.

The chromophore-bearing compound further bears at least one epoxide-reactive group. In particular, said epoxide-reactive group may correspond to -X-H wherein X is as defined above for the photoinitiator of the invention. More particularly, the epoxide-reactive group -X-H may correspond to one of the following groups -NHR¹, -OH, -SH, *-C(=O)-OH, or ^{∗}-C(=O)-NHR¹, wherein R¹ is independently H, alkyl or aryl.

In particular, component a) may comprise, consist of or consist essentially of at least one compound according to the following formula (II):

Q-R³-X-H (II)

wherein Q, X and R³ are as defined above.

All the preferred embodiments for Q, X and R³ described above for the photoinitiator of the invention, equally apply to component a).

More particularly, the chromophore bearing component a) may comprise, consist of or consist essentially of at least one compound according to formula (II-a) to (II-f): 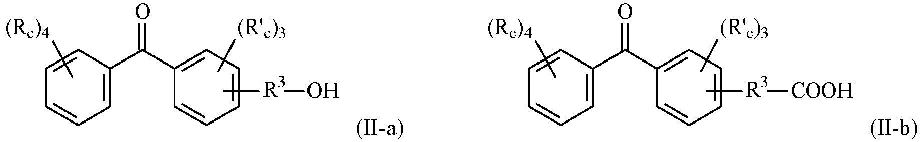 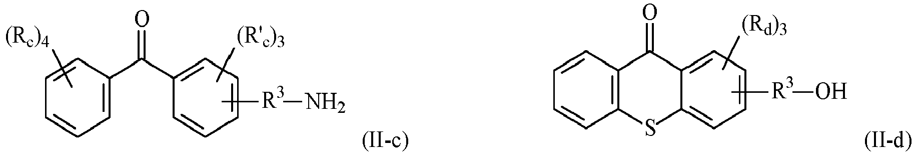 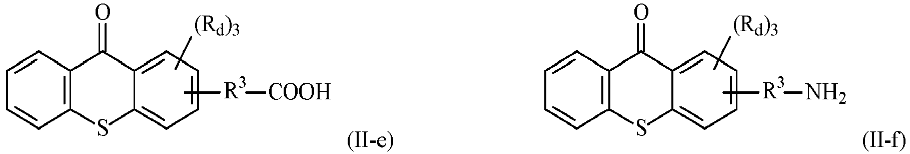 wherein
R³ is selected from a direct bond, an alkylene of formula (C-1) as defined above, an oxyalkylene of formula (C-2) as defined above and an aminoalkylene of formula (C-5) as defined above;
each R_{c} is independently H, alkyl, Ar, -S-Ar or -O-Ar wherein Ar is aryl, in particular H, methyl, Ph, -S-Ph or -O-Ph wherein Ph is phenyl;
each R'_{c} is independently H or alkyl, in particular H or methyl;
each R_{d} is independently H, halogen, alkoxy, alkyl or -C(=O)-Ar wherein Ar is aryl, in particular H, F, Cl, methyl, ethyl, isopropyl or -C(=O)-Ph wherein Ph is phenyl.

Even more particularly, the chromophore bearing component a) may comprise, consist of or consist essentially of a compound according to formula (II-e).

### Polyepoxide component b)

The photoinitiator of the invention is derived from the reaction of one or more polyepoxide compounds. The one or more polyepoxide compounds used to obtain the photoinitiator are herein referred to as component b).

As used herein, the term "polyepoxide compound" means a compound bearing at least two epoxide rings.

The epoxide rings may be comprised in one or more of the following groups: a glycidyl ether group, a glycidyl ester group, a glycidyl amine group, a glycidyl amide group, a cycloaliphatic epoxide group, an epoxy group derived from the epoxidation of a non-cyclic carbon-carbon double bond and combinations thereof.

The polyepoxide compound may bear two epoxide rings. In particular, the polyepoxide compound may bear two glycidyl ether groups, two glycidyl ester groups or two epoxy groups derived from the epoxidation of a non-cyclic carbon-carbon double bond.

The polyepoxide compound may bear three epoxide rings. In particular, the polyepoxide compound may bear three glycidyl ether groups.

The polyepoxide compound may bear four epoxide rings. In particular, the polyepoxide compound may bear four glycidyl ether groups.

In particular, component b) may comprise, consist of or consist essentially of at least one compound according to the following formula (III): 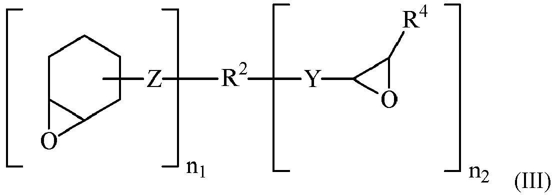 wherein
n₁ is 0, 1, 2, 3 or 4;
n₂ is 0, 1, 2, 3 or 4;
the sum n₁ + n₂ is equal to 1, 2, 3 or 4;
each Y is independently a direct bond, #-O-CH₂-, #-C(=O)-O-CH₂-, #-NR⁶-CH₂- or #-C(=O)-NR⁷-CH₂-;
each Z is independently a direct bond, -CH₂-O-C(=O))-# or -C(=O)-O-#;
R² is a direct bond or a linker;
each R⁴ is independently H, an optionally substituted alkyl or an optionally substituted alkenyl;
each R⁶ and R⁷ is independently H, alkyl, aryl or a glycidyl group according to the following formula: 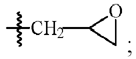
the symbol # represents a point of attachment to R².

All the preferred embodiments for n₁, n₂, Y, Z, R² and R⁴ described above for the photoinitiator of the invention, equally apply to component b).

More particularly, component b) may comprise, consist of or consist essentially of at least one compound according to one of the following formulae (III-a) to (III-n): 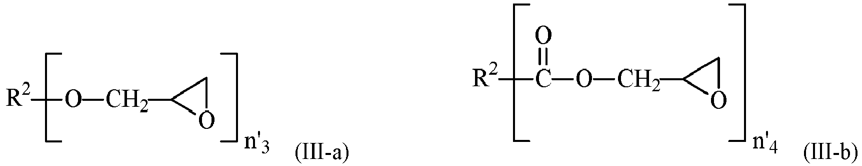 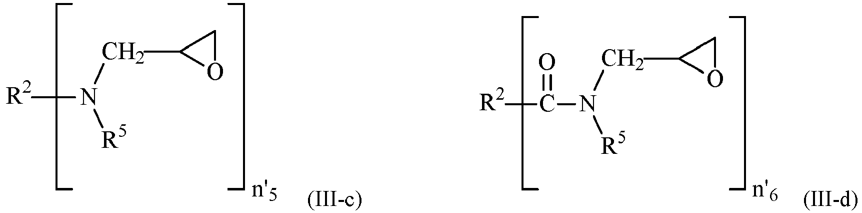 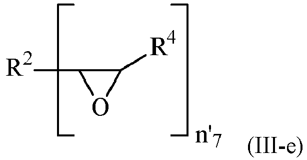 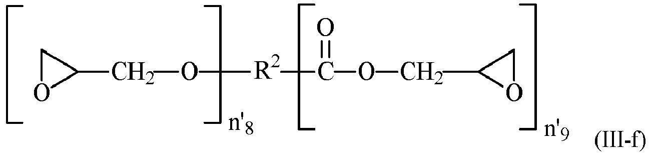 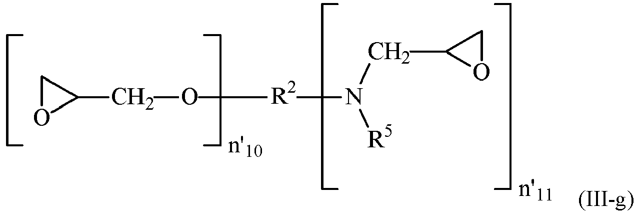 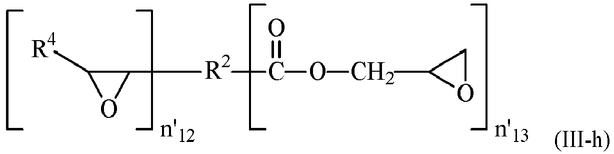 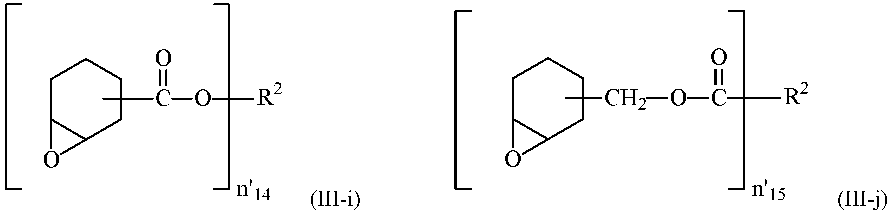 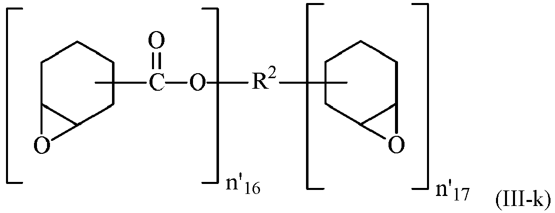 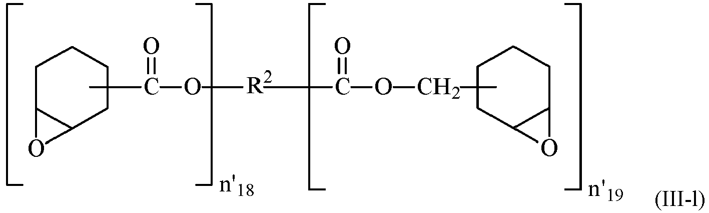 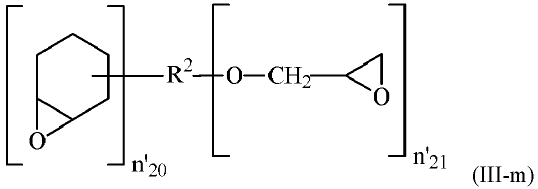 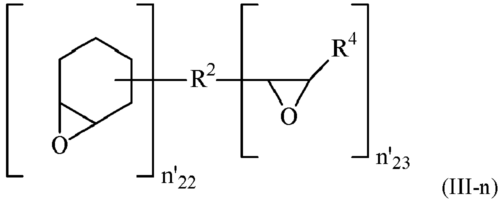 wherein
R², R⁴ and R⁵ are as defined herein;
n'₃, n'₄, n'₇, n'₁₄ and n'₁₅ are independently 2, 3 or 4, preferably 2 or 3;
n'₅ and n'₆ are independently 1 or 2, preferably 2;
n'₈ is 1, 2, or 3 preferably 2;
n'₉ is 1, 2 or 3, preferably 1;
n'₁₀, n'₁₁, n'₁₆, n'₁₇, n'₁₈, n'₁₉, n'₂₀, n'₂₁, n'₂₂ and n'₂₃ are independently 1 or 2, preferably 1;
n'₁₂ and n'₁₃ are independently 1, 2 or 3, preferably 1.

Even more particularly, component b) may comprise, consist of, or consist essentially of at least one compound selected from, but not limited to, diglycidyl ether, 1,2,3,4-diepoxybutane, 1,2,4,5-diepoxypentane, 1,2,5,6-diepoxyhexane, 1,2,7,8-diepoxyoctane, 1,2,9,10-diepoxydecane, ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, glycerol triglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, hydrogenated bisphenol A diglycidyl ether, hydrogenated bisphenol B diglycidyl ether, hydrogenated bisphenol F diglycidyl ether, hydrogenated bisphenol S diglycidyl ether, bisphenol A diglycidyl ether, bisphenol AP diglycidyl ether, bisphenol AF diglycidyl ether, bisphenol B diglycidyl ether, bisphenol BP diglycidyl ether, bisphenol C diglycidyl ether, bisphenol C2 diglycidyl ether, bisphenol F diglycidyl ether, bisphenol G diglycidyl ether, bisphenol M diglycidyl ether, bisphenol S diglycidyl ether, bisphenol P diglycidyl ether, bisphenol PH diglycidyl ether, bisphenol TMC diglycidyl ether, bisphenol Z diglycidyl ether, dinitrobisphenol A diglycidyl ether, tetrabromobisphenol A diglycidyl ether, diglycidyl phthalate, diglycidyl terephthalate, diglycidyl isophthalate, 4-(diglycidylamino)phenyl glycidyl ether, 4,4'-methylenebis(N,N-diglycidylaniline), 4-[4-[bis(oxiran-2-ylmethyl)amino]phenoxy]-N,N-bis(oxiran-2-ylmethyl)anilin, 4-(2-(4-(bis(oxiran-2-ylmethyl)amino)phenoxy)ethoxy)-N,N-bis(oxiran-2-ylmethyl)benzenamine, limonene dioxide (4-vinyl-1-cyclohexene diepoxide), an epoxidized vegetable oil, triglycidyl isocyanurate, or combinations thereof.

More particularly still, component b) may comprise, consist of, or consist essentially of at least one compound selected from 1,2,7,8-diepoxyoctane, trimethylolpropane triglycidyl ether and 1,4-butanediol diglycidyl ether.

### Uses

The photoinitiator of the present invention may be used as a photoinitiating system in a radiation curable composition, in particular in a UV or LED-curable composition.

As used herein, "UV curable composition" means a composition that is at least partially cured by exposure to UV light emitted by a mercury light source, in particular a mercury-vapor lamp, and "LED-curable composition" means a composition that is at least partially cured by exposure to UV light emitted by a LED light source, in particular a LED light source having an emission band in the range of 365-420 nm.

Other curing methods may be combined with UV light, such as heating,
The photoinitiator of the present invention may be used in a photopolymerization reaction. The photopolymerization reaction may be used to photopolymerize (e.g. cure) one or more ethylenically unsaturated compounds. As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate (including cyanoacrylate), methacrylate, acrylamide, methacrylamide, styrene, maleate, fumarate, itaconate, allyl, propenyl, vinyl and combinations thereof, preferably selected from acrylate, methacrylate and vinyl, more preferably selected from acrylate and methacrylate. The carbon-carbon double bonds of a phenyl ring are not considered as polymerizable carbon-carbon double bonds.

In one embodiment, the ethylenically unsaturated compound comprises a (meth)acrylate-functionalized compound, in particular a (meth)acrylate-functionalized compound selected from a (meth)acrylate-functionalized monomer, a (meth)acrylate-functionalized oligomer, an amine-modified acrylate and mixtures thereof.

The photoinitiator of the present invention may be used to obtain a photoreactive oligomer. The photoreactive oligomer may be obtained by reacting the photoinitiator of the invention with at least one compound bearing at least two hydroxy-reactive groups (i.e. groups that can react with the hydroxy groups of the photoinitiator). Examples of suitable hydroxyl-reactive groups include isocyanate groups and carboxylic acid groups and derivatives thereof (i.e. esters and anhydrides). For example, the photoinitiator of the present invention could be used to obtain a photoreactive urethane oligomer, i.e. by using the photoinitiator as part of or all of the polyol component that reacts with a polyisocyanate component to provide a urethane oligomer.

A urethane oligomer having photoreactive moieties in its backbone may be referred as a photoreactive urethane oligomer. In particular, the photoreactive urethane oligomer may be a photoreactive urethane (meth)acrylate oligomer. The photoreactive urethane (meth)acrylate oligomer may be obtained by reacting:
a) a polyol component comprising at least one photoinitiator of formula (I) according to the present invention and optionally one or more other polyols;
b) a polyisocyanate component; and
c) a hydroxyl-functional (meth)acrylate component.

The photoreactive urethane oligomer may be used in combination with one or more ethylenically unsaturated compound as defined above to provide a curable composition. In particular, the curable composition may be a coating composition, an ink composition, a varnish composition, an encapsulation or potting composition, a 3D-printing composition, a molding composition, a sealant composition, an adhesive composition, a nail polish composition or a dental composition.

A curable composition comprising a photoreactive urethane oligomer based on the photoinitiator of the present invention may advantageously not require an additional photoinitiator. The cured product obtained by curing such a curable composition may exhibit a reduced amount of extractables since in comparison with a composition comprising a conventional urethane oligomer and an additional photoinitiator.

The photoinitiator of the present invention could be used to obtain a photoreactive polyester oligomer, i.e. by using the photoinitiator as part of or all of the polyol component that reacts with a polyacid component to provide a polyester oligomer.

The photoinitiator of the present invention may be used to obtain a polymerizable photoinitiator, i.e. by transforming at least part of the hydroxyl groups of the photoinitiator into (meth)acrylate groups by reaction with a (meth)acrylating agent such as (meth)acrylic acid, (meth)acrylic anhydride or (meth)acryloyl chloride.

### Aspects of the invention

The invention may be as defined in the following Aspects:
Aspect 1. A photoinitiator according to the following formula (I): 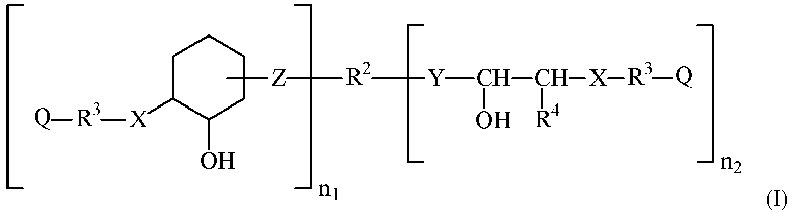 wherein
   n₁ is 0, 1, 2, 3 or 4;
   n₂ is 0, 1, 2, 3 or 4;
   the sum n₁ + n₂ is equal to 1, 2, 3 or 4;
   each Q is independently a chromophore moiety;
   each X is independently -NR¹-, -O-, -S-, *-C(=O)-O-, or ^{∗}-C(=O)-NR¹-;
   each Y is independently a direct bond, #-O-CH₂-, #-C(=O)-O-CH₂-, #-NR⁵-CH₂- or #-C(=O)-NR⁵-CH₂-;
   each Z is independently a direct bond, -CH₂-O-C(=O))-# or -C(=O)-O-#;
   each R¹ is independently H, alkyl or aryl;
   R² is a direct bond or a linker;
   each R³ is independently a direct bond or a linker;
   each R⁴ is independently H, an optionally substituted alkyl or an optionally substituted alkenyl;
   each R⁵ is independently H, alkyl, aryl or a group according to the following formula:

      -CH₂-CH(OH)-CH₂-X-R³-Q

      wherein Q, X and R³ are as defined above;
   the symbol * represents a point of attachment to R³;
   the symbol # represents a point of attachment to R².
Aspect 2. The photoinitiator according to Aspect 1, wherein n₁ is 0, 1 or 2, more particularly n₁ is 0.
Aspect 3. The photoinitiator according to Aspect 1 or 2, wherein n₂ is 2, 3 or 4, more particularly n₂ is 2 or 3.
Aspect 4. The photoinitiator according to any one of Aspects 1 to 3, wherein the sum n₁ + n₂ is equal to 2, 3 or 4, more particularly the sum n₁ + n₂ is equal to 2 or 3.
Aspect 5. The photoinitiator according to any one of Aspects 1 to 4, wherein the photoinitiator corresponds to one of the following formulae (I-a) to (I-n): 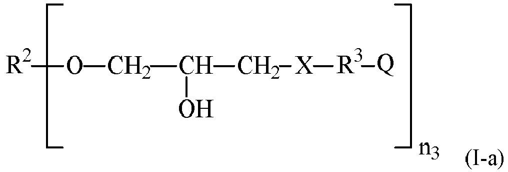 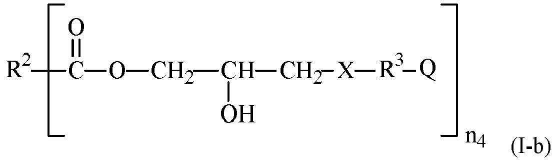 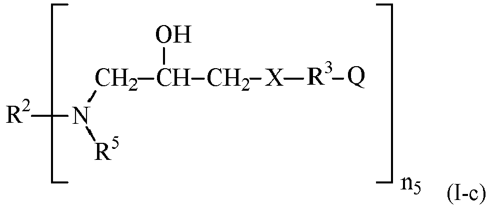 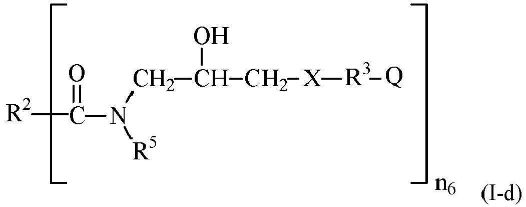 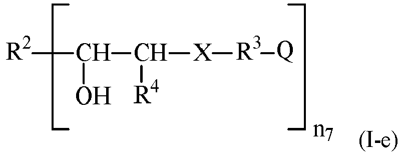 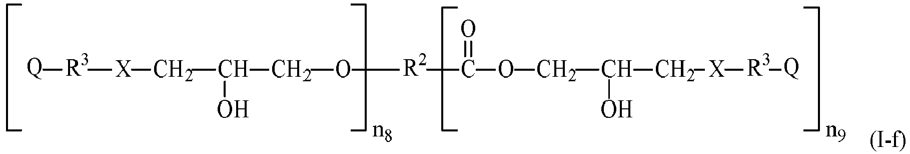 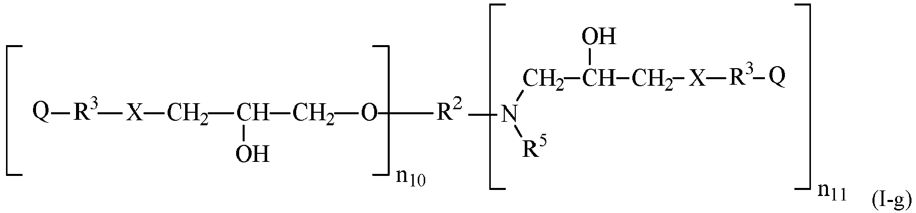 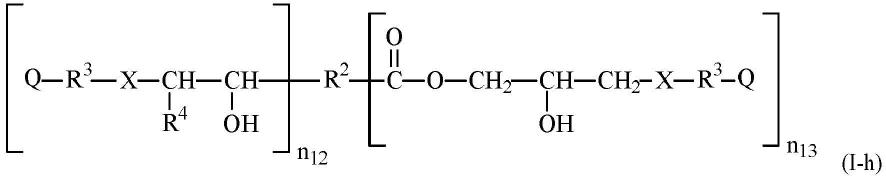 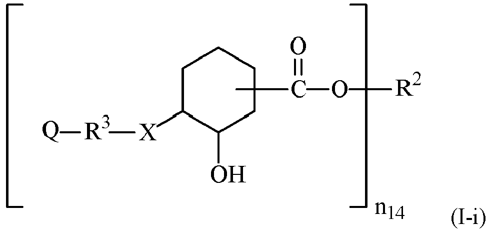 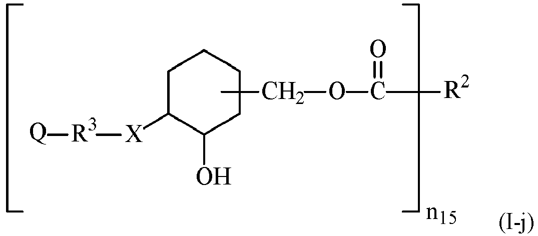 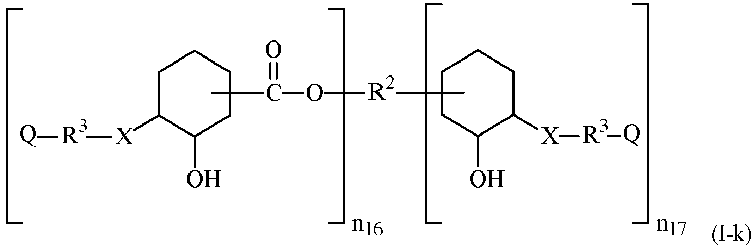 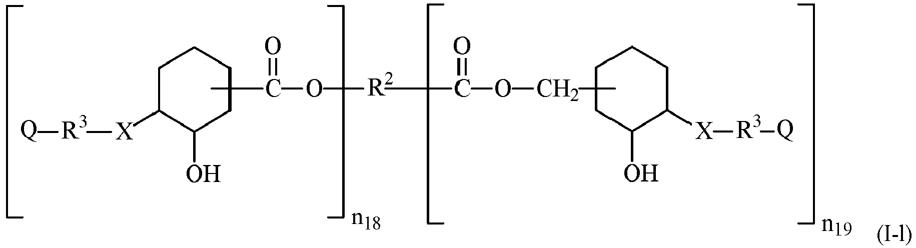 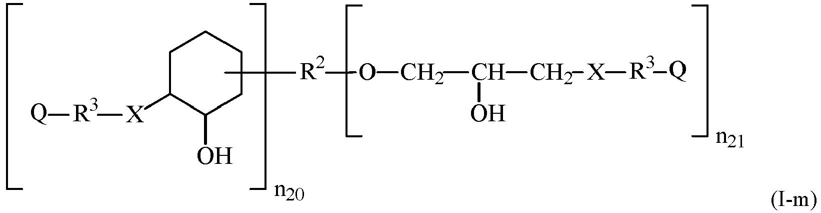 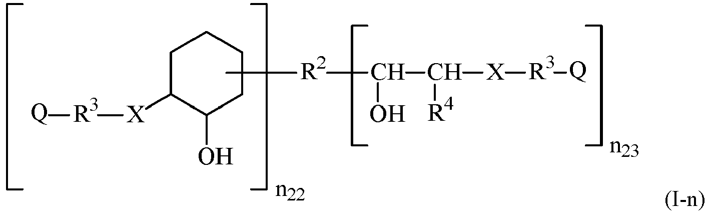 wherein
   Q, X, R², R³, R⁴ and R⁵ are as defined in Aspect 1;
   n₃, n₄, n₇, n₁₄ and n₁₅ are independently 2, 3 or 4, preferably 2 or 3;
   n₅ and n₆ are independently 1 or 2, preferably 2;
   n₈ is 1, 2, or 3 preferably 2;
   n₉ is 1, 2 or 3, preferably 1;
   n₁₀, n₁₁, n₁₆, n₁₇, n₁₈, n₁₉, n₂₀, n₂₁, n₂₂ and n₂₃ are independently 1 or 2, preferably 1;
   n₁₂ and n₁₃ are independently 1, 2 or 3, preferably 1.
Aspect 6. The photoinitiator according to Aspect 5, wherein the photoinitiator corresponds to one of formulae (I-a), (I-b), (I-c), (I-e), (I-f), (I-g) and (I-h), in particular to one of formulae (I-a), (I-b) (I-c) and (I-e).
Aspect 7. The photoinitiator according to any one of Aspects 1 to 6, wherein the photoinitiator comprises 2, 3 or 4 chromophore moieties Q.
Aspect 8. The photoinitiator according to any one of Aspects 1 to 7, wherein each chromophore moiety Q independently comprises a benzophenone moiety, a thioxanthone moiety, an anthraquinone moiety, a xanthone moiety or an acridone moiety; in particular each chromophore moiety Q is independently a benzophenone moiety according to the following formula (A) or a thioxanthone, anthraquinone, xanthone or acridone moiety according to the following formula (B): 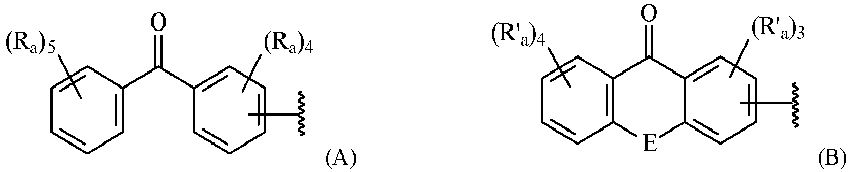 wherein
   E is S, O, C(=O) or NR, in particular S;
   R is H, an optionally substituted alkyl or an optionally substituted aryl;
   each Rₐ and R'ₐ is independently H, F, Cl, Br, I, -OR_{b}, -SR_{b}, -N(R_{b})₂, -NOz, -CN, -C(=O)R_{b}, -O-C(=O)R_{b}, -C(=O)OR_{b}, -C(=O)N(R_{b})₂, -NR_{b}-C(=O)-R_{b}, -SO₂-N(R_{b})₂, or an optionally substituted group selected from alkyl, heteroatom-containing alkyl, cycloalkyl, heterocycloalkyl, alkenyl, alkynyl, aryl, aralkyl, alkaryl and heteroaryl;
   two adjacent Rₐ or R'ₐ groups may form, with the carbon atoms to which they are attached, a 5 to 8 membered ring;
   each R_{b} is independently H or an optionally substituted group selected from alkyl, heteroatom-containing alkyl, cycloalkyl, heterocycloalkyl, alkenyl, alkynyl, aryl, aralkyl, alkaryl and heteroaryl.
Aspect 9. The photoinitiator according to Aspect 8, wherein each chromophore moiety Q is a benzophenone moiety according to the following formula (A1): 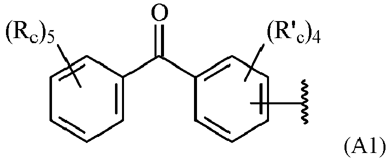 wherein
   each R_{c} is independently H, alkyl, Ar, -S-Ar or -O-Ar wherein Ar is aryl, in particular H, methyl, Ph, -S-Ph or -O-Ph wherein Ph is phenyl;
   each R'_{c} is independently H or alkyl, in particular H or methyl.
Aspect 10. The photoinitiator according to Aspect 8, wherein each chromophore moiety Q is a thioxanthone moiety according to the following formula (B1): 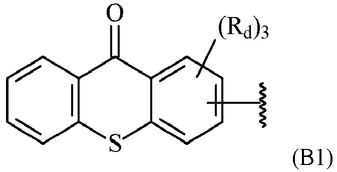 wherein
   each R_{d} is independently H, halogen, alkoxy, alkyl or -C(=O)-Ar wherein Ar is aryl, in particular H, F, Cl, methyl, ethyl, isopropyl or -C(=O)-Ph wherein Ph is phenyl.
Aspect 11. The photoinitiator according to any one of Aspects 1 to 10, wherein each R³ is independently a direct bond or a divalent linker comprising 1 to 15, preferably 1 to 10, carbon atoms, and optionally one or more heteroatoms, such as O, N or S.
Aspect 12. The photoinitiator according to any one of Aspects 1 to 11, wherein each R³ is independently a direct bond, a C1-C6 alkylene, a C1-C6 oxyalkylene, a C1-C6 alkenylene, a C1-C6 thioalkylene, a C1-C6 ketoalkylene or a C1-C6 aminoalkylene; in particular each R³ is independently a direct bond, an alkylene of formula (C-1), an oxyalkylene of formula (C-2), a thioalkylene of formula (C-3), a ketoalkylene of formula (C-4), or an aminoalkylene of formula (C-5)

   •-(CRₘR'ₘ)_{f}-§ (C-1)

   •-O-(CRₒR'ₒ)_{g}-§ (C-2)

   •-S-(CRₚR'ₚ)ₕ-§ (C-3)

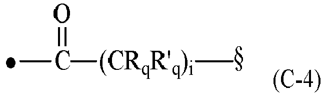

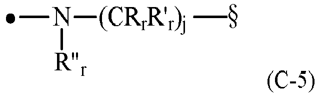

   wherein
   each Rₘ, R'ₘ, Rₒ, R'ₒ, Rₚ, R'ₚ, R_{q}, R'_{q} Rᵣ and R'ᵣ is independently H or an optionally substituted alkyl, in particular H;
   R"ᵣ is H or an optionally substituted alkyl;
   f is 1, 2, 3, 4, 5 or 6, in particular 1 or 2;
   g is 1, 2, 3, 4, 5 or 6, in particular 1 or 2;
   h is 1, 2, 3, 4, 5 or 6, in particular 1 or 2;
   i is 1, 2, 3, 4 or 5, in particular 1;
   j is 1, 2, 3, 4, 5 or 6, in particular 1 or 2;
   the symbol ● represents a point of attachment to the Q moiety;
   the symbol § represents a point of attachment to the X moiety.
Aspect 13. The photoinitiator according to Aspect 12, wherein each R³ is independently a direct bond, an alkylene of formula (C-1) or an oxyalkylene of formula (C-2), in particular each R³ is a direct bond, an alkylene of formula (C-1) or an oxyalkylene of formula (C-2).
Aspect 14. The photoinitiator according to any one of Aspects 1 to 13, wherein each X is independently -NR¹-, -O- or *-C(=O)-O-; in particular -NR¹- or *-C(=O)-O-.
Aspect 15. The photoinitiator according to any one of Aspects 1 to 14, wherein each R² is independently a direct bond, an aromatic linker or an aliphatic linker; in particular R² is an aliphatic linker.
Aspect 16. The photoinitiator according to any one of Aspects 1 to 15, wherein each R² is independently a direct bond or a linker other than a polyether linker; in particular each R² is independently a direct bond or a non-polymeric linker.
Aspect 17. The photoinitiator according to any one of Aspects 1 to 16, wherein each R² is independently a direct bond or a linker selected from the group consisting of alkylene, heteroatom-containing alkylene, alkenylene, heteroatom-containing alkenylene, cycloalkylene, heterocycloalkylene, arylene, heteroarylene, and combinations thereof;
   in particular R² is a direct bond or a linker selected from the group consisting of alkylene, heteroatom-containing alkylene, cycloalkylene, arylene and combinations thereof.
Aspect 18. The photoinitiator according to any one of Aspects 1 to 17, wherein each R⁴ is H.
Aspect 19. The photoinitiator according to any one of Aspects 6 to 8 and 10 to 18, wherein the photoinitiator is according to one of the following formulae (I-a), (I-b), (I-c) or (I-e), preferably one of formula (I-a) or (I-e): 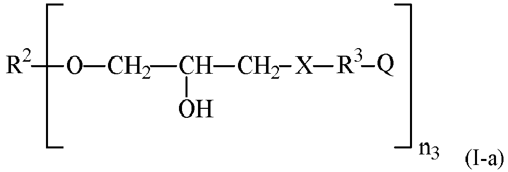 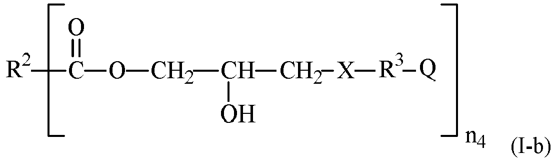 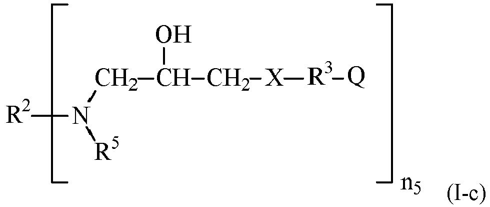 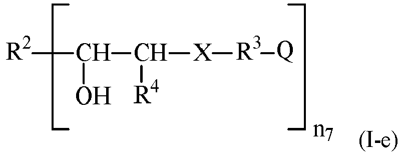 wherein
   n₃, n₄, n₅, and n₇ are independently 2, 3 or 4, preferably 2 or 3;
   each chromophore moiety Q is according to formula (A) or (B) as defined in Aspect 8, preferably each chromophore moiety Q is according to formula (A1) as defined in Aspect 9 or according to formula (B1) as defined in Aspect 10;
   each X is independently -NR¹-, -O- or *-C(=O)-O-, preferably-NR'- or *-C(=O)-O-;
   each R¹ is independently H or alkyl;
   each R² is independently an alkylene or a heteroatom-containing alkylene;
   each R³ is independently a direct bond, an alkylene of formula (C-1) as defined in Aspect 12, an oxyalkylene of formula (C-2) as defined in Aspect 12 or an aminoalkylene of formula (C-5) as defined in Aspect 12, preferably a direct bond, an alkylene of formula (C-1) or oxyalkylene of formula (C-2);
   each R⁴ is H;
   each R⁵ is independently H, alkyl, aryl or -CH₂-CH(OH)-CH₂-X-R³-Q wherein X, R³ and Q are as defined above;
   the symbol * represents a point of attachment to R³.
Aspect 20. The photoinitiator according to Aspect 1, wherein the photoinitiator is according to one of the following formulae (I-1) to (I-9): 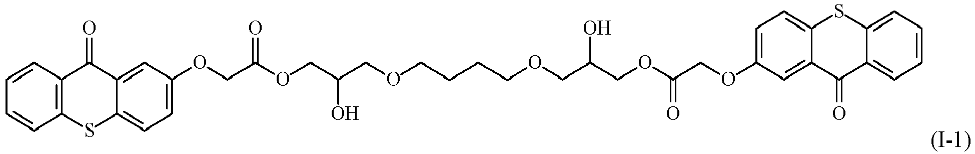 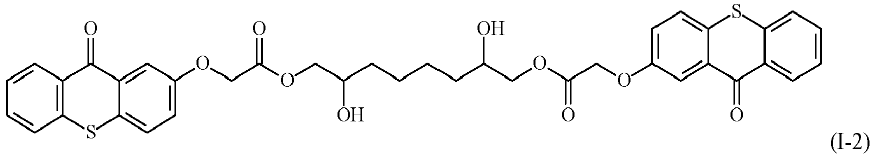 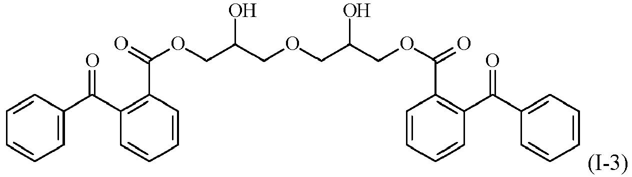 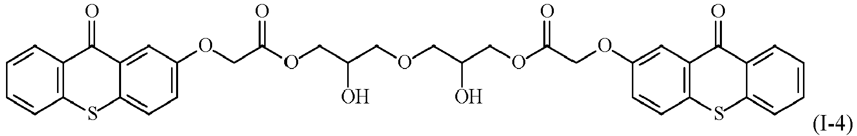 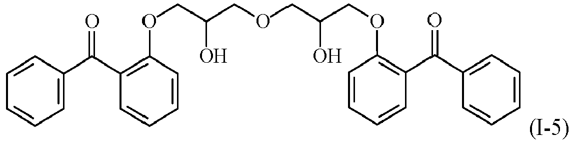 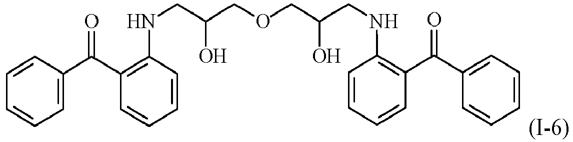 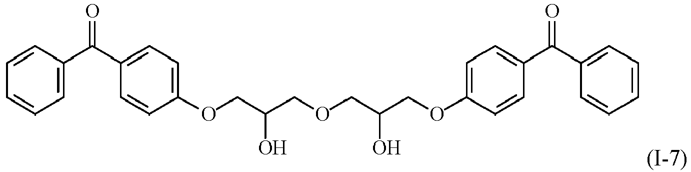 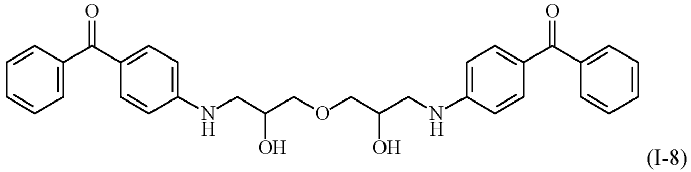 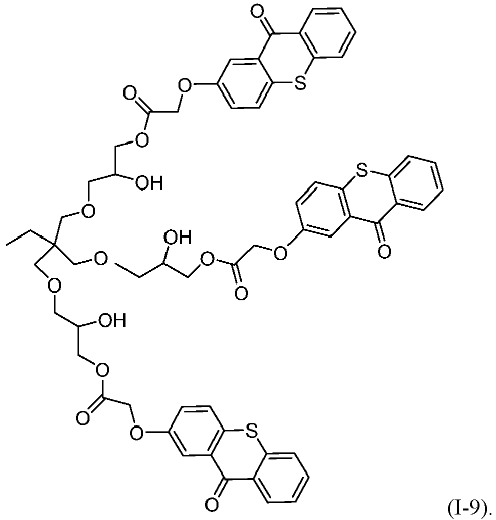
Aspect 21. A method of preparing a photoinitiator by reacting a chromophore-bearing component a) with a polyepoxide component b); wherein component a) comprises at least one compound bearing at least one chromophore moiety and at least one epoxide-reactive group; and component b) comprises at least one compound bearing at least two epoxide rings.
Aspect 22. The method according to Aspect 21, wherein the photoinitiator obtained with the method corresponds to the photoinitiator of formula (I) as defined in any one of Aspects 1 to 20.
Aspect 23. The method according to Aspect 21 or 22, wherein component a) comprises at least one compound according to the following formula (II):

   Q-R³-X-H (II)

   wherein
   Q is a chromophore moiety;
   X is -NR¹-, -O-, -S-, *-C(=O)-O-, or ^{∗}-C(=O)-NR¹-; and
   R³ is a direct bond or a linker;
   the symbol * represents a point of attachment to R³.
Aspect 24. The method according to any one of Aspects 21 to 23, wherein component a) comprises at least one compound according to one of the following formulae (II-a) to (II-f): 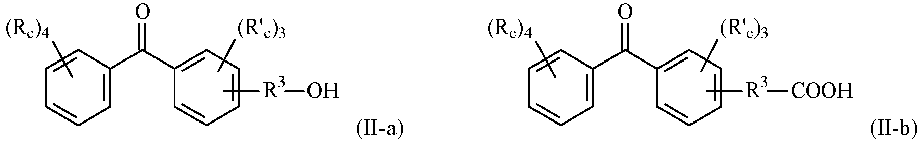 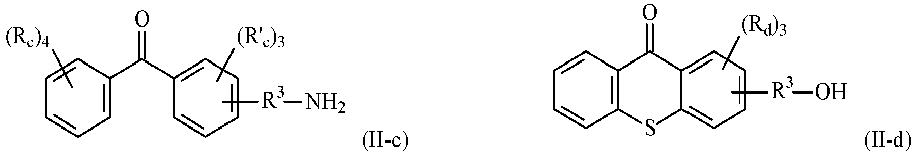 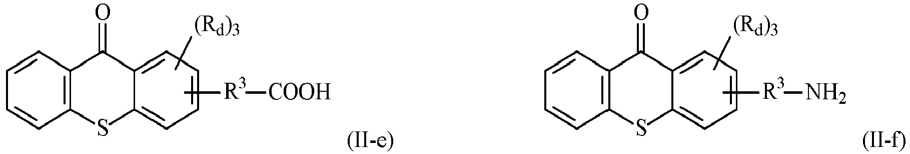 wherein
   R³ is selected from a direct bond, an alkylene of formula (C-1) as defined in Aspect 12, an oxyalkylene of formula (C-2) as defined in Aspect 12 and an aminoalkylene of formula (C-5) as defined in Aspect 12;
   each R_{c} is independently H, alkyl, Ar, -S-Ar or -O-Ar wherein Ar is aryl, in particular H, methyl, Ph, -S-Ph or -O-Ph wherein Ph is phenyl;
   each R'_{c} is independently H or alkyl, in particular H or methyl;
   each R_{d} is independently H, halogen, alkoxy, alkyl or -C(=O)-Ar wherein Ar is aryl, in particular H, F, Cl, methyl, ethyl, isopropyl or -C(=O)-Ph wherein Ph is phenyl.
Aspect 25. The method according to Aspect 24, wherein component a) comprises at least one compound according to formula (II-e).
Aspect 26. The method according to any one of Aspects 21 to 25, wherein component b) comprises at least one compound according to the following formula (III): 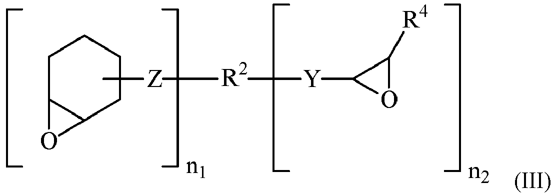 wherein
   n₁ is 0, 1, 2, 3 or 4;
   n₂ is 0, 1, 2, 3 or 4;
   the sum n₁ + n₂ is equal to 1, 2, 3 or 4;
   each Y is independently a direct bond, #-O-CH₂-, #-C(=O)-O-CH₂-, #-NR⁶-CH₂- or #-C(=O)-NR⁷-CH₂-;
   each Z is independently a direct bond, -CH₂-O-C(=O))-# or -C(=O)-O-#;
   R² is a direct bond or a linker;
   each R⁴ is independently H, an optionally substituted alkyl or an optionally substituted alkenyl;
   each R⁶ and R⁷ is independently H, alkyl, aryl or a glycidyl group of formula 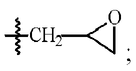
   the symbol # represents a point of attachment to R².
Aspect 27. The method according to any one of Aspects 21 to 26, wherein component b) comprises at least one compound according to one of the following formulae (III-a) to (III-n): 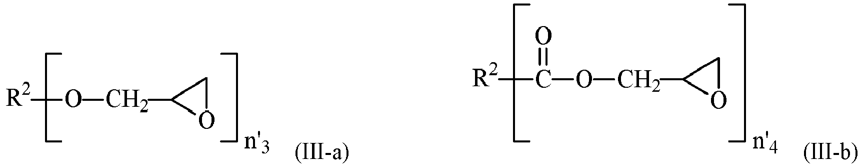 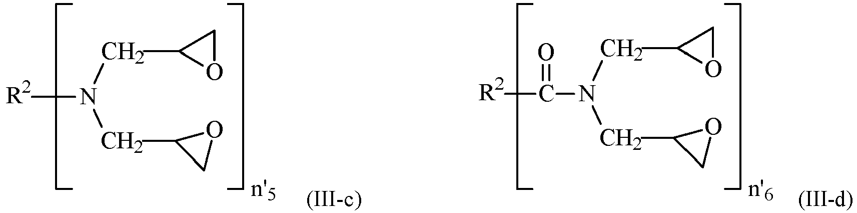 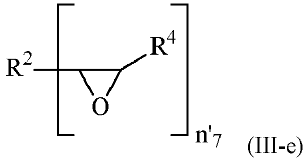 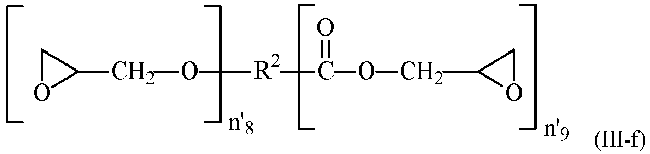 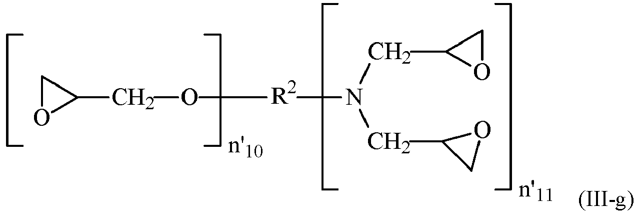 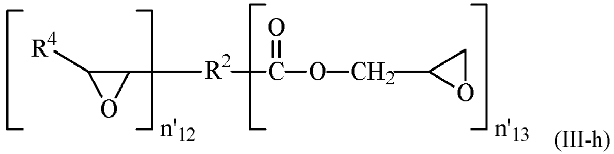 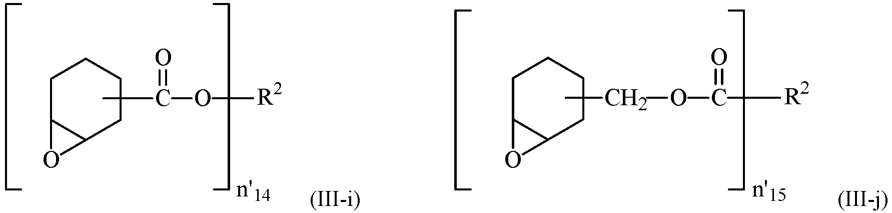 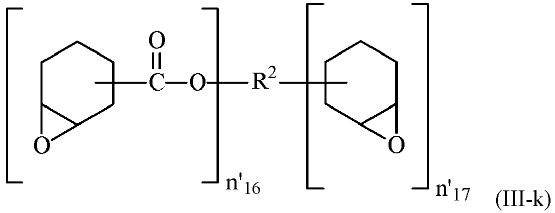 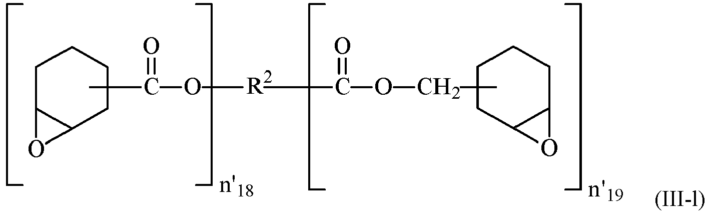 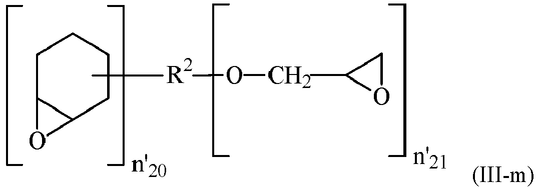 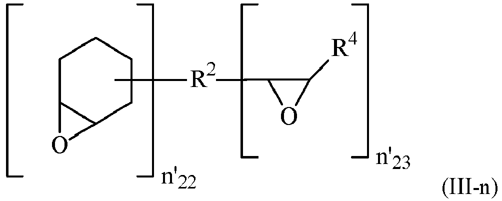 wherein
   R² and R⁴ are as defined in Aspect 26 ;
   n'₃, n'₄, n'₇, n'₁₄ and n'₁₅ are independently 2, 3 or 4, preferably 2 or 3;
   n'₅ and n'₆ are independently 1 or 2, preferably 2;
   n'₈ is 1, 2, or 3 preferably 2;
   n'₉ is 1, 2 or 3, preferably 1;
   n'₁₀, n'₁₆, n'₁₇, n'₁₈, n'₁₉, n'₂₀, n'₂₁, n'₂₂ and n'₂₃ are independently 1 or 2, preferably 1;
   n'₁₂ and n'₁₃ are independently 1, 2 or 3, preferably 1.
Aspect 28. The method according to any one of Aspects 21 to 27, wherein component b) comprises at least one compound selected from diglycidyl ether, 1,2,3,4-diepoxybutane, 1,2,4,5-diepoxypentane, 1,2,5,6-diepoxyhexane, 1,2,7,8-diepoxyoctane, 1,2,9,10-diepoxydecane, ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propanediol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, glycerol triglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, hydrogenated bisphenol A diglycidyl ether, hydrogenated bisphenol B diglycidyl ether, hydrogenated bisphenol F diglycidyl ether, hydrogenated bisphenol S diglycidyl ether, bisphenol A diglycidyl ether, bisphenol AP diglycidyl ether, bisphenol AF diglycidyl ether, bisphenol B diglycidyl ether, bisphenol BP diglycidyl ether, bisphenol C diglycidyl ether, bisphenol C2 diglycidyl ether, bisphenol F diglycidyl ether, bisphenol G diglycidyl ether, bisphenol M diglycidyl ether, bisphenol S diglycidyl ether, bisphenol P diglycidyl ether, bisphenol PH diglycidyl ether, bisphenol TMC diglycidyl ether, bisphenol Z diglycidyl ether, dinitrobisphenol A diglycidyl ether, tetrabromobisphenol A diglycidyl ether, diglycidyl phthalate, diglycidyl terephthalate, diglycidyl isophthalate, 4-(diglycidylamino)phenyl glycidyl ether, 4,4'-methylenebis(N,N-diglycidylaniline), 4-[4-[bis(oxiran-2-ylmethyl)amino]phenoxy]-N,N-bis(oxiran-2-ylmethyl)anilin, 4-(2-(4-(bis(oxiran-2-ylmethyl)amino)phenoxy)ethoxy)-N,N-bis(oxiran-2-ylmethyl)benzenamine, limonene dioxide (4-vinyl-1-cyclohexene diepoxide), an epoxidized vegetable oil, triglycidyl isocyanurate, or combinations thereof.
Aspect 29. The method according to any one of Aspects 21 to 28, wherein component b) comprises at least one compound selected from 1,2,7,8-diepoxyoctane, trimethylolpropane triglycidyl ether and 1,4-butanediol diglycidyl ether.
Aspect 30. The method according to any of Aspects 21 to 29, wherein the reaction between component a) and component b) is carried out in the presence of at least one catalyst.
Aspect 31. The method according to any of Aspects 21 to 30, wherein the reaction between component a) and component b) is carried out at a temperature of from 50 to 150°C, in particular 70 to 130°C.
Aspect 32. A use of the photoinitiator according to any one of Aspects 1 to 20, as a photoinitiating system in a radiation curable composition.
Aspect 33. A use of the photoinitiator according to any one of Aspects 1 to 20, in a photopolymerization reaction.
Aspect 34. The use according to Aspect 32, wherein the photoinitiator is used to photopolymerize one or more ethylenically unsaturated compounds.
Aspect 35. A use of the photoinitiator according to any one of Aspects 1 to 20, to obtain a photoreactive oligomer, in particular a photoreactive urethane oligomer, more particularly a photoreactive urethane (meth)acrylate oligomer.

The invention will be illustrated in more detail with reference to the following Examples, but it should be understood that the invention is not deemed to be limited thereto.

### EXAMPLES

### Example 1:

A thioxanthone (TX)-based diol of formula (I-o) was prepared according to the following scheme: 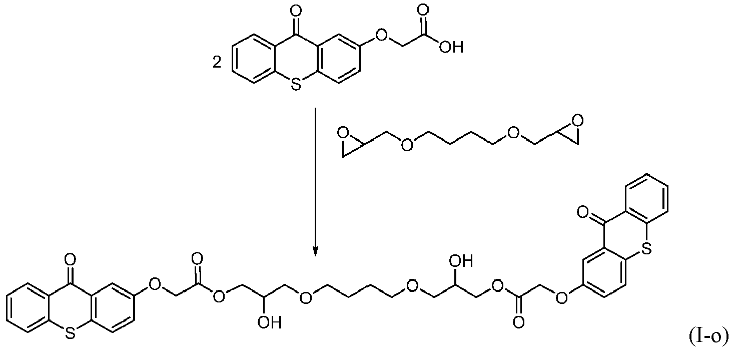 2-(Carboxymethoxy)thioxanthone (31.8 gram, 0.1054 mole, Lambson); 1,4-butanediol diglycidyl ether (GE-21 from CVC, 15 gram, 0.1289 moles of epoxide rings); benzyltriethylammonium chloride (BTEAC, 0.14g, 0.3 wt. %, Aldrich) as catalyst and 50 wt% cyclopentanone as process solvent were combined in a three-neck flask equipped with mechanical stirring under nitrogen, and heated to 125°C. The reaction was then run at 125°C until shut down criteria were reached at acid value (AV) < 2 mg KOH/gm and epoxy value (EV) < 2 mg KOH/gm (about 3 hours). The AV or EV was adjusted by adding more CMTX or 1,4-butanediol diglycidyl ether as the reaction proceeded as needed. The final diol product (I-o) was a dark brown liquid and exhibited acceptable FT-IR and ¹H NMR spectral features.

### Example 2:

A thioxanthone (TX)-based triol of formula (I-p) was prepared according to the following scheme:

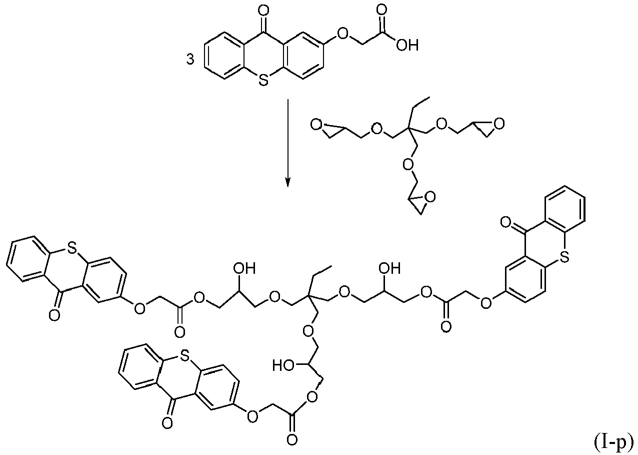

2-(Carboxymethoxy)thioxanthone (CMTX, 35.2 gram, 0.1167 mole), trimethylolpropane triglycidyl ether (18.7 gram, 0.136 mole of epoxide rings, GE-30 from CVC), benzyltriethylammonium chloride (BTEAC, 0.14 g, 0.26 wt. % ) as catalyst and cyclopentanone (54 g) as process solvent were mixed and heated to 130 °C. The reaction was then run at 130 °C until shut down criteria were reached at acid value (AV) < 2 mg KOH/gm and epoxy value (EV) < 2 mg KOH/gm (about 3 hours). The AV or EV was adjusted by adding more CMTX or trimethylolpropane triglycidyl ether as the reaction proceeded as needed. The final product (I-p) was a dark brown liquid exhibiting expected FT-IR and ¹H NMR spectral characteristics.

### Example 3:

A thioxanthone (TX)-based triol of formula (I-q) was prepared according to the following scheme:

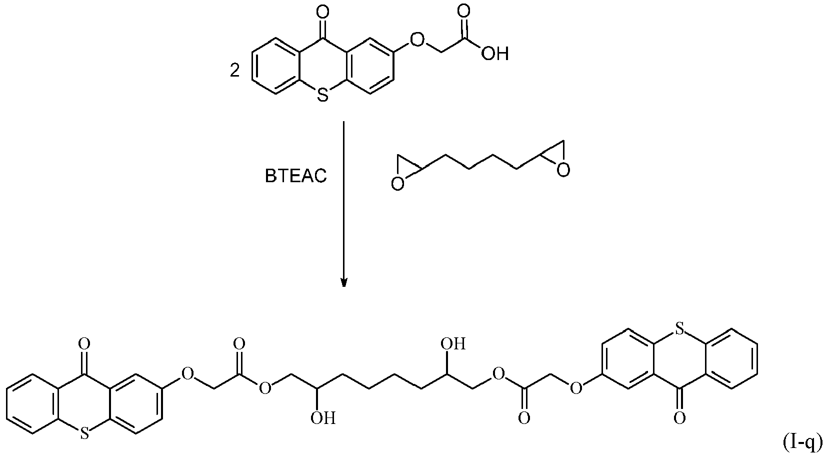

2-(Carboxymethoxy)thioxanthone (CMTX, 31.8 gram, 0.1054 mole); 1,2,7,8-diepoxyoctane (7.49 gram, ca. 0.105 moles of epoxide rings); benzyltriethylammonium chloride (BTEAC, 0.14g, 0.3 wt% ) as catalyst and cyclopentanone (40 g, to produce an approximately 50 wt.% solution) as process solvent were combined in a three-neck flask equipped with mechanical stirring under nitrogen, and heated to 125°C. The reaction was run at 125°C until shut down criteria were reached at AV < 2 mg KOH/gm and EV < 2 mg KOH/gm are attained (about 3 hours). The AV or EV was adjusted by adding more CMTX or 1,2,7,8-diepoxyoctane as the reaction proceeded as needed. The final product (I-q) exhibits expected FT-IR and ¹H NMR spectral characteristics.

Within this specification embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

The foregoing description of various forms of the invention has been presented for purposes of illustration and description. It is not intended to be exhaustive or to limit the invention to the precise forms disclosed. Numerous modifications or variations are possible in light of the above teachings. The forms discussed were chosen and described to produce the best illustration of the principles of the invention and its practical application to thereby enable one of ordinary skill in the art to utilize the invention in various forms and with various modifications as are suited to the particular use contemplated. All such modifications and variations are within the scope of the invention as determined by the appended claims when interpreted in accordance with the breadth to which they are fairly, legally, and equitably entitled.

## Claims

1. A photoinitiator according to the following formula (I): 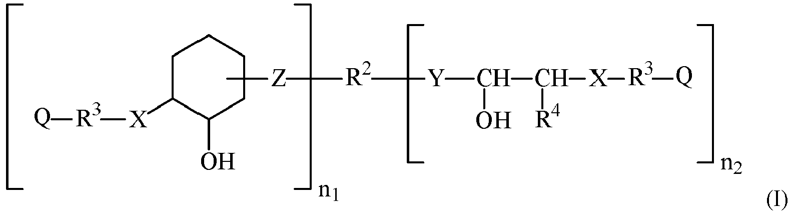 wherein
n₁ is 0, 1, 2, 3 or 4;
n₂ is 0, 1, 2, 3 or 4;
the sum n₁ + n₂ is equal to 1, 2, 3 or 4;
each Q is independently a chromophore moiety;
each X is independently -NR¹-, -O-, -S-, *-C(=O)-O-, or ^{∗}-C(=O)-NR¹-;
each Y is independently a direct bond, #-O-CH₂-, #-C(=O)-O-CH₂-, #-NR⁵-CH₂- or #-C(=O)-NR⁵-CH₂-;
each Z is independently a direct bond, -CH₂-O-C(=O))-# or -C(=O)-O-#;
each R¹ is independently H, alkyl or aryl;
R² is a direct bond or a linker;
each R³ is independently a direct bond or a linker;
each R⁴ is independently H, an optionally substituted alkyl or an optionally substituted alkenyl;
each R⁵ is independently H, alkyl, aryl or a group according to the following formula:
-CH₂-CH(OH)-CH₂-X-R³-Q
wherein Q, X and R³ are as defined above;
the symbol * represents a point of attachment to R³;
the symbol # represents a point of attachment to R².

2. The photoinitiator according to claim 1, wherein the photoinitiator corresponds to one of the following formulae (I-a) to (I-n): 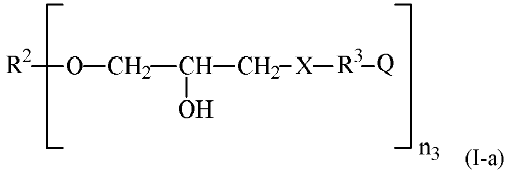 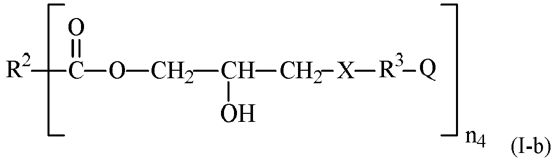 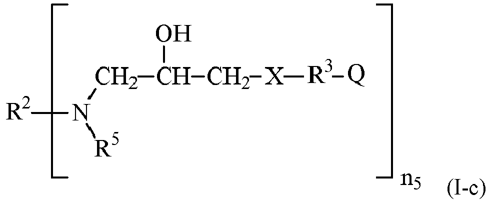 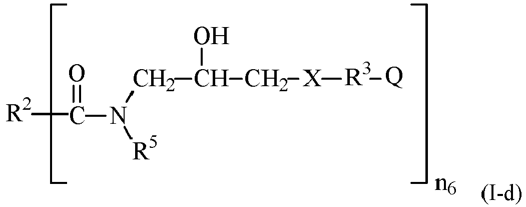 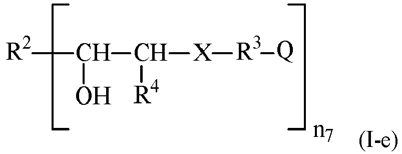 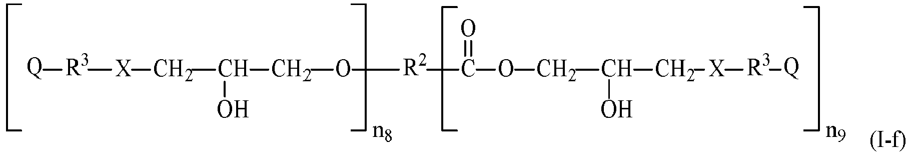 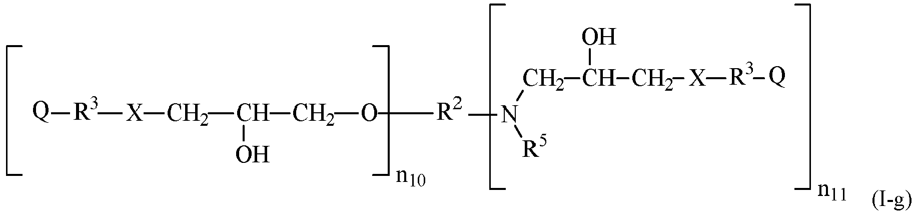 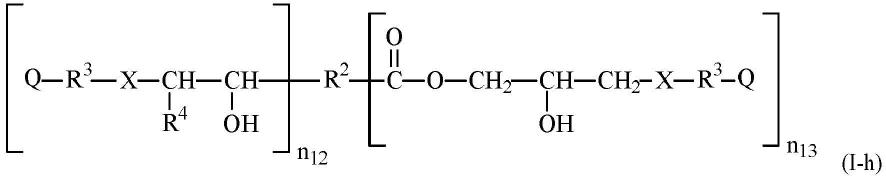 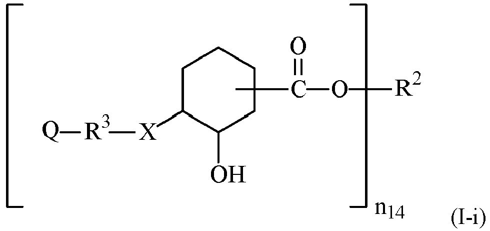 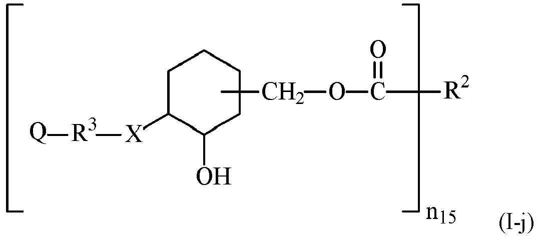 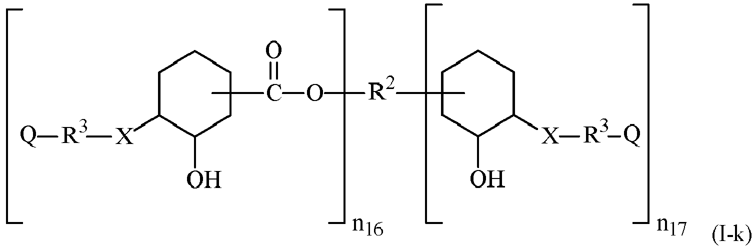 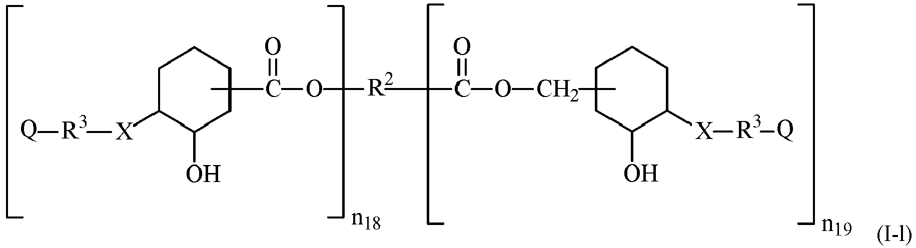 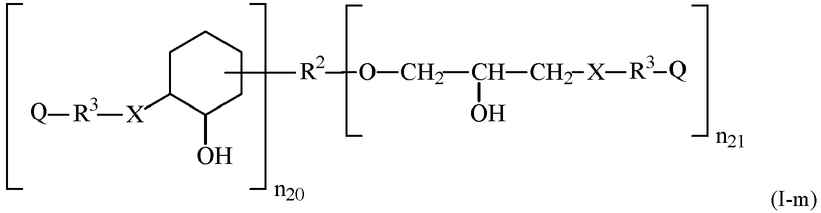 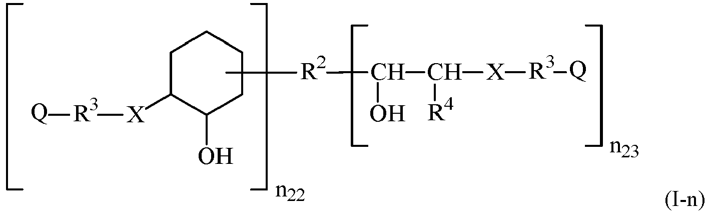 wherein
Q, X, R², R³, R⁴ and R⁵ are as defined in claim 1;
n₃, n₄, n₇, n₁₄ and n₁₅ are independently 2, 3 or 4, preferably 2 or 3;
n₅ and n₆ are independently 1 or 2, preferably 2;
n₈ is 1, 2, or 3 preferably 2;
n₉ is 1, 2 or 3, preferably 1;
n₁₀, n₁₁, n₁₆, n₁₇, n₁₈, n₁₉, n₂₀, n₂₁, n₂₂ and n₂₃ are independently 1 or 2, preferably 1;
n₁₂ and n₁₃ are independently 1, 2 or 3, preferably 1.

3. The photoinitiator according to claim 1 or 2, wherein each chromophore moiety Q independently comprises a benzophenone moiety, a thioxanthone moiety, an anthraquinone moiety, a xanthone moiety or an acridone moiety; in particular each chromophore moiety Q is independently a benzophenone moiety according to the following formula (A) or a thioxanthone, anthraquinone, xanthone or acridone moiety according to the following formula (B): 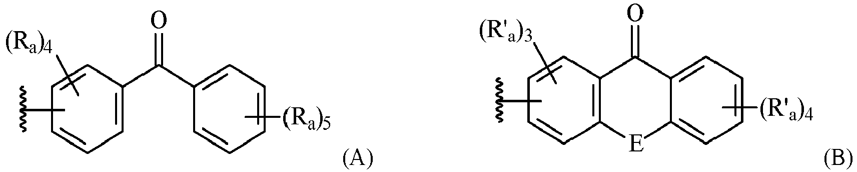 wherein
E is S, O, C(=O) or NR, in particular S;
R is H, an optionally substituted alkyl or an optionally substituted aryl;
each Rₐ and R'ₐ is independently H, F, Cl, Br, I, -OR_{b}, -SR_{b}, -N(R_{b})₂, -NOz, -CN, -C(=O)R_{b}, -O-C(=O)R_{b}, -C(=O)OR_{b}, -C(=O)N(R_{b})₂, -NR_{b}-C(=O)-R_{b}, -SO₂-N(R_{b})₂ or an optionally substituted group selected from alkyl, heteroatom-containing alkyl, cycloalkyl, heterocycloalkyl, alkenyl, alkynyl, aryl, aralkyl, alkaryl and heteroaryl;
two adjacent Rₐ or R'ₐ groups may form, with the carbon atoms to which they are attached, a 5 to 8 membered ring;
each R_{b} is independently H or an optionally substituted group selected from alkyl, heteroatom-containing alkyl, cycloalkyl, heterocycloalkyl, alkenyl, alkynyl, aryl, aralkyl, alkaryl and heteroaryl.

4. The photoinitiator according to claim 3, wherein each chromophore moiety Q is a benzophenone moiety according to the following formula (A1) or a thioxanthone moiety according to the following formula (B1): 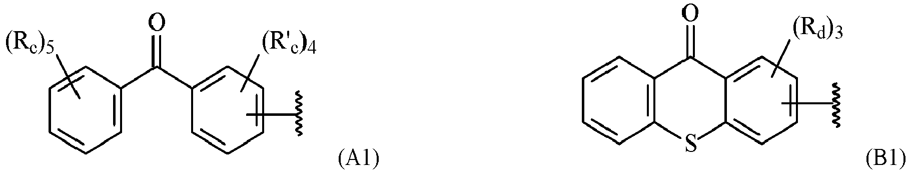 wherein
each R_{c} is independently H, alkyl, Ar, -S-Ar or -O-Ar wherein Ar is aryl, in particular H, methyl, Ph, -S-Ph or -O-Ph wherein Ph is phenyl;
each R'_{c} is independently H or alkyl, in particular H or methyl;
each R_{d} is independently H, halogen, alkoxy, alkyl or -C(=O)-Ar wherein Ar is aryl, in particular H, F, Cl, methyl, ethyl, isopropyl or -C(=O)-Ph wherein Ph is phenyl.

5. The photoinitiator according to any one of claims 1 to 4, wherein each R³ is independently a direct bond, a C1-C6 alkylene, a C1-C6 oxyalkylene, a C1-C6 alkenylene, a C1-C6 thioalkylene, a C1-C6 ketoalkylene or a C1-C6 aminoalkylene; in particular each R³ is independently a bond, an alkylene of formula (C-1), an oxyalkylene of formula (C-2), a thioalkylene of formula (C-3), a ketoalkylene of formula (C-4) or an aminoalkylene of formula (C-5):
•-(CRₘR'ₘ)_{f}-§ (C-1)
•-O-(CRₒR'ₒ)_{g}-§ (C-2)
•-S-(CRₚR'ₚ)ₕ-§ (C-3) 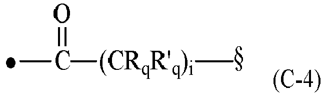 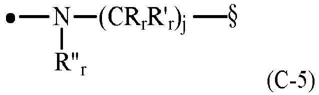
wherein
each Rₘ, R'ₘ, Rₒ, R'ₒ, Rₚ, R'ₚ, R_{q}, R'_{q}, Rᵣ and R'ᵣ is independently H or an optionally substituted alkyl, in particular H;
R"ᵣ is H or an optionally substituted alkyl;
f is 1, 2, 3, 4, 5 or 6, in particular 1 or 2;
g is 1, 2, 3, 4, 5 or 6, in particular 1 or 2;
h is 1, 2, 3, 4, 5 or 6, in particular 1 or 2;
i is 1, 2, 3, 4 or 5, in particular 1;
j is 1, 2, 3, 4, 5 or 6, in particular 1 or 2;
the symbol ● represents a point of attachment to the Q moiety;
the symbol § represents a point of attachment to the X moiety.

6. The photoinitiator according to any one of claims 1 to 13, wherein each X is independently -NR¹-, -O-, -S-, or *-C(=O)-O-; in particular -NR¹-, -O- or *-C(=O)-O-, more particularly -NR¹- or *-C(=O)-O-.

7. The photoinitiator according to any one of claims 1 to 6, wherein R² is a direct bond or a linker selected from the group consisting of alkylene, heteroatom-containing alkylene, alkenylene, heteroatom-containing alkenylene, cycloalkylene, heterocycloalkylene, arylene, heteroarylene, and combinations thereof;
in particular R² is a direct bond or a linker selected from the group consisting of alkylene, heteroatom-containing alkylene, cycloalkylene, arylene and combinations thereof.

8. The photoinitiator according to any one of claims 2 to 7, wherein the photoinitiator is according to one of the following formulae (I-a), (I-b), (I-c) or (I-e), preferably one of formula (I-a) or (I-e): 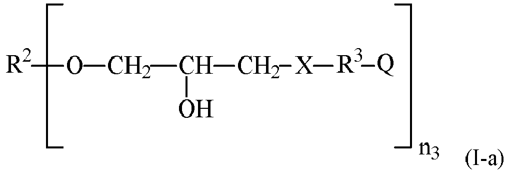 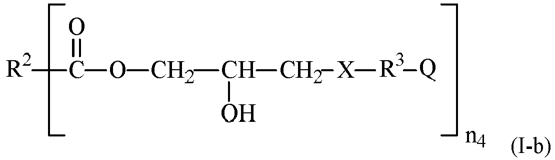 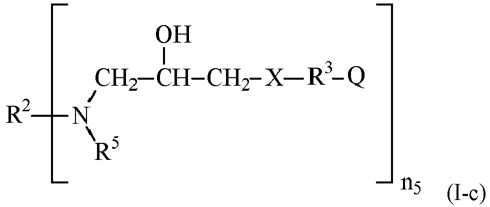 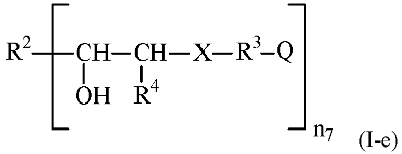 wherein
n₃, n₄ and n₇ are independently 2, 3 or 4, preferably 2 or 3;
each chromophore moiety Q is according to formula (A) or (B) as defined in claim 3, preferably each chromophore moiety Q is according to formula (A1) or (B1) as defined in claim 4;
each X is independently -NR¹-, -O- or *-C(=O)-O-, preferably -NR¹- or *-C(=O)-O-;
each R¹ is independently H or alkyl;
each R² is independently an alkylene or a heteroatom-containing alkylene;
each R³ is independently a direct bond, an alkylene of formula (C-1), an oxyalkylene of formula (C-2) or an aminoalkylene of formula (C-5) as defined in claim 5, preferably a direct bond, an alkylene of formula (C-1) or an oxyalkylene of formula (C-2);
each R⁴ is H;
the symbol * represents a point of attachment to R³.

9. A method of preparing a photoinitiator by reacting a chromophore-bearing component a) with a polyepoxide component b), wherein
component a) comprises at least one compound bearing at least one chromophore moiety and at least one epoxide-reactive group;
component b) comprises at least one compound bearing at least two epoxide rings.

10. The method according to claim 9, wherein the photoinitiator corresponds to the photoinitiator of formula (I) as defined in any one of claims 1 to 8.

11. The method according to claim 9 or 10, wherein component a) comprises at least one compound according to the following formula (II):
Q-R³-X-H (II)
wherein
Q is a chromophore moiety;
X is -NR¹-, -O-, -S-, *-C(=O)-O-, or ^{∗}-C(=O)-NR¹-; and
R³ is a direct bond or a linker;
the symbol * represents a point of attachment to R³.

12. The method according to any one of claims 9 to 11, wherein component b) comprises at least one compound according to the following formula (III): 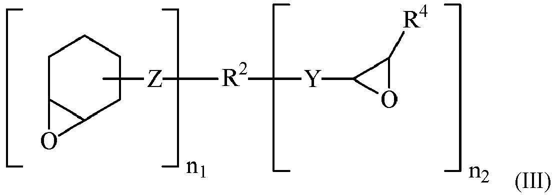 wherein
n₁ is 0, 1, 2, 3 or 4;
n₂ is 0, 1, 2, 3 or 4;
the sum n₁ + n₂ is equal to 1, 2, 3 or 4;
each Y is independently a direct bond, #-O-CH₂-, #-C(=O)-O-CH₂-, #-NR⁶-CH₂- or #-C(=O)-NR⁷-CH₂-;
each Z is independently a direct bond, -CH₂-O-C(=O))-# or -C(=O)-O-#;
R² is a direct bond or a linker;
each R⁴ is independently H, an optionally substituted alkyl or an optionally substituted alkenyl;
each R⁶ and R⁷ is independently H, alkyl, aryl or a glycidyl group of formula 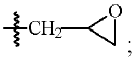
the symbol # represents a point of attachment to R².

13. A use of the photoinitiator according to any one of claims 1 to 8, as a photoinitiating system in a radiation curable composition.

14. A use of the photoinitiator according to any one of claims 1 to 8, in a photopolymerization reaction.

15. A use of the photoinitiator according to any one of claims 1 to 8, to obtain a photoreactive oligomer, in particular a photoreactive urethane oligomer, more particularly a photoreactive urethane (meth)acrylate oligomer.
